# EUROPEAN PATENT APPLICATION

(11) **EP 4 183 410 A1**
(43) Date of publication of application: **24.05.2023**
(21) Application number: 21842214.5
(22) Date of filing: 16.07.2021
(51) Int. Cl.: A61K 39/12, A61P 31/14, A61K 9/00, A61K 47/26, A61K 47/36, A61M 37/00

(54) **PREVENTIVE AGENT FOR JAPANESE ENCEPHALITIS AND JAPANESE ENCEPHALITIS VACCINE**

(30) Priority: 17.07.2020 JP 2020122621
(71) Applicant: FUJIFILM Corporation, Tokyo 106-8620 (JP); National University Corporation Hokkaido University, Sapporo-shi, Hokkaido 060-0808 (JP)
(72) Inventor: OYAMADA, Takayoshi, Ashigarakami-gun, Kanagawa 258-8577 (JP); SHIMADA, Toshio, Ashigarakami-gun, Kanagawa 258-8577 (JP); KAKITA, Kosuke, Ashigarakami-gun, Kanagawa 258-8577 (JP); IWATA, Hiroaki, Sapporo-shi, Hokkaido 060-0808 (JP)
(74) Representative: Klunker IP Patentanwälte PartG mbB
(86) International application number: PCT/JP2021/026729
(87) International publication number: WO 2022/014695

(57) **Abstract**

It is an object of the present invention to provide a Japanese encephalitis preventive agent and a Japanese encephalitis vaccine agent, which are capable of imparting sufficient immunity to humans, even if these agents are used at a lower dose than the dose of a Japanese encephalitis vaccine for subcutaneous injection, or at a smaller number of administrations than the number of administrations of a Japanese encephalitis vaccine for subcutaneous injection. According to the present invention, provided is a Japanese encephalitis preventive agent, comprising a microneedle array having a sheet portion and a plurality of needle portions present on the upper surface of the sheet portion, wherein the aforementioned needle portions contain or carry an inactivated Japanese encephalitis virus.

## Description

### Technical Field

The present invention relates to a Japanese encephalitis preventive agent and a Japanese encephalitis vaccine agent, each comprising a microneedle array that contains or carries an inactivated Japanese encephalitis virus.

### Background Art

As a drug administration method for administering an appropriate amount of drug and achieving sufficient medicinal effects, a method of painlessly injecting a drug into the skin, in which a microneedle array comprising microneedles (needle portions) formed with a high aspect ratio is used and the microneedles are penetrated into a stratum corneum barrier layer, has attracted attention. For example, an autolytic microneedle array comprising, as a base material, a substance having *in vivo* solubility, has been reported. In such an autolytic microneedle array, a drug is retained in the base material, and when the microneedles are inserted into the skin, the base material is autolyzed, so that the drug can be administered into the skin.

Patent Document 1 discloses a microneedle device comprising a substrate, microneedles disposed on the substrate, and coating layers formed on the microneedles, wherein the length of the microneedle is 300 to 500 µm, the microneedles are disposed on the substrate at a density of 28 to 80 microneedles/cm², and the coating layers have physiologically active substances. Patent Document 1 describes that the skin irritation of the microneedle device having coating layers comprising Japanese encephalitis vaccines to animals.

Patent Document 2 discloses a microneedle array having a sheet portion and a plurality of needle portions present on the upper surface of the sheet portion, wherein the needle portions comprise at least one type of an electrically neutral water-soluble polymer and a disaccharide, a Japanese encephalitis vaccine, and an electrically neutral surfactant, and the sheet portion comprises at least one type of an electrically neutral water-soluble polymer and a disaccharide.

### Prior Art Documents

### Patent Documents

Patent Document 1: International Publication WO2018/155431
Patent Document 2: International Publication WO2019/225650

### Summary of Invention

Object to be Solved by the Invention

Patent Document 1 does not describe the neutralizing antibody titer or the skin irritation of the microneedle device upon administration thereof to humans. Non-Patent Document 1 does not describe a significant difference between microneedles and an intramuscular injection in terms of neutralizing antibody titer and positive conversion percentage, and further, Non-Patent Document 1 does not contain descriptions regarding a Japanese encephalitis vaccine.

In the case of commercially available Japanese encephalitis vaccines for subcutaneous injection, two times of inoculations are required as initial immunizations with intervals of 1 to 4 weeks, and further, in a period 1 year after completion of the initial immunizations, a booster is carried out, so that basic immunity can be achieved by a total of 3 times of inoculations. Thus, a large amount of Japanese encephalitis vaccine becomes necessary. However, since it takes a longer time to produce vaccines than to produce common pharmaceutical products, vaccines may be in short supply due to unforeseen circumstances.

It is an object of the present invention to provide a Japanese encephalitis preventive agent and a Japanese encephalitis vaccine agent, which are capable of imparting sufficient immunity to humans, even if these agents are used at a lower dose than the dose of a Japanese encephalitis vaccine for subcutaneous injection, or at a smaller number of administrations than the number of administrations of a Japanese encephalitis vaccine for subcutaneous injection.

### Means for Solving the Object

As a result of intensive studies conducted directed towards achieving the aforementioned object, the present inventors have found that, by using a microneedle array having a sheet portion and a plurality of needle portions that contain or carry an inactivated Japanese encephalitis virus, even though the inactivated Japanese encephalitis virus is used at a dose of 1/4 of the dose of a Japanese encephalitis vaccine for subcutaneous injection, one administration of the inactivated Japanese encephalitis virus to a human as an initial immunization exhibits a positive conversion percentage equivalent to two subcutaneous injections thereof, and even though the inactivated Japanese encephalitis virus is used at a dose of 1/10 of the dose of a Japanese encephalitis vaccine for subcutaneous injection, two administrations of the inactivated Japanese encephalitis virus exhibit a positive conversion percentage equivalent to the subcutaneous injection. The present invention has been completed based on these findings.

Specifically, according to the present invention, the following inventions are provided.
(1) A Japanese encephalitis preventive agent, comprising a microneedle array having a sheet portion and a plurality of needle portions present on the upper surface of the sheet portion, wherein the needle portions contain or carry an inactivated Japanese encephalitis virus.
(2) The Japanese encephalitis preventive agent according to (1), wherein the inactivated Japanese encephalitis virus is administered at a dose of 0.01 µg to 2.5 µg/administration.
(3) The Japanese encephalitis preventive agent according to (1) or (2), wherein the number of administrations is once or twice.
(4) The Japanese encephalitis preventive agent according to any one of (1) to (3), wherein the inactivated Japanese encephalitis virus is administered twice at a dose of 0.01 µg to 0.22 µg/administration.
(5) The Japanese encephalitis preventive agent according to any one of (1) to (3), wherein the inactivated Japanese encephalitis virus is administered once or twice at a dose of 0.23 µg to 1 µg/administration.
(6) The Japanese encephalitis preventive agent according to any one of (1) to (5), wherein the microneedle array is an autolytic microneedle array, and the needle portions contain the inactivated Japanese encephalitis virus.
(7) The Japanese encephalitis preventive agent according to any one of (1) to (6), wherein the needle portions comprise at least one type of a water-soluble polymer and a disaccharide, an inactivated Japanese encephalitis virus, and a surfactant, and the sheet portion comprises at least one type of a water-soluble polymer and a disaccharide.
(8) The Japanese encephalitis preventive agent according to any one of (1) to (7), wherein the neutralizing antibody titer 2 weeks after the initial administration is 1.0 or more; provided that the neutralizing antibody titer is herein defined to be a common logarithm of the highest dilution rate of a serum that suppressed 50% or more of the cytopathic effect of the Japanese encephalitis virus, when 20 µL of a serum sample obtained by serially diluting a serum collected from a subject 2 weeks after administration of the Japanese encephalitis preventive agent is allowed to react with 80 pfu of Japanese encephalitis virus at 37°C and in 5% CO₂ for 1.5 hours, thereafter, 25 µL of the mixture obtained after completion of the reaction is infected into 2.0 x 10⁴ Vero cells, and thereafter, the obtained mixture are cultured at 37°C and in 5% CO₂ for 6 days.
(9) A Japanese encephalitis vaccine agent, comprising a microneedle array having a sheet portion and a plurality of needle portions present on the upper surface of the sheet portion, wherein the needle portions contain or carry an inactivated Japanese encephalitis virus.
(10) The Japanese encephalitis vaccine agent according to (9), wherein the inactivated Japanese encephalitis virus is administered at a dose of 0.01 µg to 2.5 µg/administration.
(11) The Japanese encephalitis vaccine agent according to (9) or (10), wherein the number of administrations is once or twice.
(12) The Japanese encephalitis vaccine agent according to any one of (9) to (11), wherein the inactivated Japanese encephalitis virus is administered twice at a dose of 0.01 µg to 0.22 µg/administration.
(13) The Japanese encephalitis vaccine agent according to any one of (9) to (11), wherein the inactivated Japanese encephalitis virus is administered once or twice at a dose of 0.23 µg to 1 µg/administration.
(14) The Japanese encephalitis vaccine agent according to any one of (9) to (13), wherein the microneedle array is an autolytic microneedle array, and the needle portions contain the inactivated Japanese encephalitis virus.
(15) The Japanese encephalitis vaccine agent according to any one of (9) to (14), wherein the needle portions comprise at least one type of a water-soluble polymer and a disaccharide, an inactivated Japanese encephalitis virus, and a surfactant, and the sheet portion comprises at least one type of a water-soluble polymer and a disaccharide.
(16) The Japanese encephalitis vaccine agent according to any one of (9) to (15), wherein the neutralizing antibody titer 2 weeks after the initial administration is 1.0 or more; provided that the neutralizing antibody titer is herein defined to be a common logarithm of the highest dilution rate of a serum that suppresses 50% or more of the cytopathic effect of the Japanese encephalitis virus, when 20 µL of a serum sample obtained by serially diluting a serum collected from a subject 2 weeks after administration of the Japanese encephalitis vaccine agent is allowed to react with 80 pfu of Japanese encephalitis virus at 37°C and in 5% CO₂ for 1.5 hours, thereafter, 25 µL of the mixture obtained after completion of the reaction is infected into 2.0 x 10⁴ Vero cells, and thereafter, the obtained mixture are cultured at 37°C and in 5% CO₂ for 6 days.
   (A) A method for preventing Japanese encephalitis, comprising administering to a subject, a microneedle array having a sheet portion and a plurality of needle portions present on the upper surface of the sheet portion, wherein the needle portions contain or carry an inactivated Japanese encephalitis virus.
   (B) A method for imparting immunity against Japanese encephalitis to a subject, comprising administering to the subject, a microneedle array having a sheet portion and a plurality of needle portions present on the upper surface of the sheet portion, wherein the needle portions contain or carry an inactivated Japanese encephalitis virus.
   (C) A microneedle array having a sheet portion and a plurality of needle portions present on the upper surface of the sheet portion, wherein the needle portions contain or carry an inactivated Japanese encephalitis virus, and wherein the microneedle array is for use in a treatment for preventing Japanese encephalitis.
   (D) A microneedle array having a sheet portion and a plurality of needle portions present on the upper surface of the sheet portion, wherein the needle portions contain or carry an inactivated Japanese encephalitis virus, and wherein the microneedle array is for use in a treatment for imparting immunity against Japanese encephalitis to a subject.
   (E) Use of a microneedle array having a sheet portion and a plurality of needle portions present on the upper surface of the sheet portion, wherein the needle portions contain or carry an inactivated Japanese encephalitis virus, for the production of a Japanese encephalitis preventive agent.
   (F) Use of a microneedle array having a sheet portion and a plurality of needle portions present on the upper surface of the sheet portion, wherein the needle portions contain or carry an inactivated Japanese encephalitis virus, for the production of a Japanese encephalitis vaccine agent.

### Advantageous Effects of Invention

According to the present invention, even though the present Japanese encephalitis preventive agent or Japanese encephalitis vaccine agent is used at a lower dose than the dose of a Japanese encephalitis vaccine for subcutaneous injection, or at a smaller number of administrations than the number of administrations of a Japanese encephalitis vaccine for subcutaneous injection, sufficient immunity can be imparted to humans.

### Brief Description of Drawings

[Fig.1] Fig. 1A is a perspective view of a conical microneedle; Fig. 1B is a perspective view of a pyramidal microneedle; and Fig. 1C is a cross-sectional view of conical and pyramidal microneedles.
[Fig.2] Fig. 2 is a perspective view of another shape of microneedle.
[Fig.3] Fig. 3 is a perspective view of another shape of microneedle.
[Fig.4] Fig. 4 is a cross-sectional view of the microneedles shown in Fig. 2 and Fig. 3.
[Fig.5] Fig. 5 is a perspective view of another shape of microneedle.
[Fig.6] Fig. 6 is a perspective view of another shape of microneedle.
[Fig.7] Fig. 7 is a cross-sectional view of the microneedles shown in Fig. 5 and Fig. 6.
[Fig.8] Fig. 8 is a cross-sectional view of another shape of microneedle, in which inclination (angle) of the side of the needle portion is continuously changed.
[Fig.9] Figs. 9A to C are process charts showing a method for producing a mold.
[Fig. 10] Fig. 10 is an enlarged view of a mold.
[Fig.11] Fig. 11 is a cross-sectional view showing another shape of mold.
[Fig.12] Figs. 12A to C are outline views showing a step of filling a solution containing an inactivated Japanese encephalitis virus into a mold.
[Fig. 13] Fig. 13 is a perspective view showing a tip of a nozzle.
[Fig. 14] Fig. 14 is a partially enlarged view showing a nozzle tip during filling of a solution, and a mold.
[Fig. 15] Fig. 15 is a partially enlarged view showing a nozzle tip during movement, and a mold.
[Fig. 16] Figs. 16A to D are explanatory views showing a step of forming another microneedle array.
[Fig.17] Figs. 17A to C are explanatory views showing a step of forming another microneedle array.
[Fig.18] Fig. 18 is an explanatory view showing a peeling step.
[Fig.19] Fig. 19 is an explanatory view showing another peeling step.
[Fig.20] Fig. 20 is an explanatory view showing a microneedle array.
[Fig.21] Figs. 21(A) and (B) are a plan view and a side view of an original plate, respectively.
[Fig.22] Fig. 22 is a schematic view of a filling device used in the Examples.
[Fig.23] Fig. 23 shows the results obtained by measuring the applied dose of an inactivated Japanese encephalitis virus in individual subjects.

### Embodiments of Carrying out the Invention

Hereinafter, the embodiments of the present invention will be described in detail.

In the present description, the phrase "to contain an inactivated Japanese encephalitis virus" is used to mean that a Japanese encephalitis vaccine contains an inactivated Japanese encephalitis virus in an amount sufficient for the Japanese encephalitis vaccine to exhibit the effects thereof, when the vaccine is applied onto the surface of a body via puncture. On the other hand, the phrase "not to contain an inactivated Japanese encephalitis virus" is used to mean that a Japanese encephalitis vaccine does not contain an inactivated Japanese encephalitis virus in an amount sufficient for the Japanese encephalitis vaccine to exhibit the effects thereof. The range of the amount of the inactivated Japanese encephalitis virus that is not contained in the vaccine includes a range from the case of containing no inactivated Japanese encephalitis virus at all, to the amount of the inactivated Japanese encephalitis virus with which no effects are exhibited.

In the present description, the amount of an inactivated Japanese encephalitis virus in a preparation means the amount of the inactivated Japanese encephalitis virus contained as a protein content.

The present invention relates to a Japanese encephalitis preventive agent and a Japanese encephalitis vaccine agent, each comprising a microneedle array having a sheet portion and a plurality of needle portions present on the upper surface of the sheet portion, wherein the needle portions contain or carry an inactivated Japanese encephalitis virus.

According to the present invention, when compared with a Japanese encephalitis vaccine for subcutaneous injection, the number of administrations of an inactivated Japanese encephalitis virus in initial immunization can be reduced, and the single dose of the inactivated Japanese encephalitis virus can be reduced. Further, when compared with a subcutaneous injection, a neutralizing antibody titer equivalent to or greater than the subcutaneous injection can be obtained from the present invention.

The Japanese encephalitis preventive agent means a medicament used for preventing Japanese encephalitis.

The Japanese encephalitis vaccine agent means a medicament, which comprises an inactivated Japanese encephalitis virus as an active ingredient (vaccine), and is used to impart immunity against Japanese encephalitis to a subject.

### [Administration of Japanese encephalitis preventive agent and Japanese encephalitis vaccine agent]

When the Japanese encephalitis preventive agent and the Japanese encephalitis vaccine agent according to the present invention are administered to a human subject, an inactivated Japanese encephalitis virus is administered to the human subject, preferably at a dose of 0.01 µg to 2.5 µg/administration, more preferably at a dose of 0.01 µg to 2 µg/administration, and further preferably at a dose of 0.01 µg to 1 µg/administration.

The number of administrations of the Japanese encephalitis preventive agent and the Japanese encephalitis vaccine agent according to the present invention is not particularly limited, and it is preferably once or twice.

When the Japanese encephalitis preventive agent and the Japanese encephalitis vaccine agent according to the present invention are administered to a human subject, for example, it is preferable that an inactivated Japanese encephalitis virus is administered twice at a dose of 0.01 µg to 0.22 µg/administration, or that the inactivated Japanese encephalitis virus is administered once or twice at a dose of 0.23 µg to 1 µg/administration.

When the Japanese encephalitis preventive agent or the Japanese encephalitis vaccine agent according to the present invention is used, the neutralizing antibody titer 2 weeks after the initial administration is preferably 1.0 or more, more preferably 1.1 or more, and further preferably 1.2 or more. The neutralizing antibody titer 2 weeks after the initial administration may also be 1.5 or more, 1.8 or more, 2.0 or more, or 2.4 or more.

The Japanese encephalitis preventive agent or the Japanese encephalitis vaccine agent according to the present invention can be used at such a dose that the neutralizing antibody titer 2 weeks after the initial administration becomes 1.0 or more (or the neutralizing antibody titer may also be 1.1 or more, 1.2 or more, 1.5 or more, 1.8 or more, 2.0 or more, or 2.4 or more). The neutralizing antibody titer is measured by the following method. That is, 20 µL of a serum sample obtained by serially diluting a serum collected from a subject 2 weeks after administration of the Japanese encephalitis preventive agent or the Japanese encephalitis vaccine agent is allowed to react with 80 pfu of Japanese encephalitis virus at 37°C and in 5% CO₂ for 1.5 hours. Thereafter, 25 µL of the mixture obtained after completion of the reaction is infected into 2.0 x 10⁴ Vero cells, and thereafter, the obtained mixture are cultured at 37°C and in 5% CO₂ for 6 days. Upon the above-described culture, a common logarithm of the highest dilution rate of the serum that suppresses 50% or more of the cytopathic effect of the Japanese encephalitis virus is defined to be a neutralizing antibody titer.

### [Configuration of microneedle array]

The microneedle array of the present invention is a microneedle array having a sheet portion and a plurality of needle portions present on the upper surface of the sheet portion, wherein the needle portions contain or carry an inactivated Japanese encephalitis virus. In the present invention, the term "a plurality of" means one or more.

In the microneedle array, a plurality of needle portions are arranged in an array state on the upper surface side of a sheet portion. The needle portions are preferably arranged on the supper surface side of the sheet portion. The needle portions may be directly arranged on the upper surface of the sheet portion, or the needle portions may also be arranged on the upper surface of a member disposed on the upper surface of the sheet portion (preferably a member having the shape of a frustum portion).

The microneedle array has the following embodiments.

In a first embodiment, the needle portions contain an inactivated Japanese encephalitis virus, and when the needle portions are inserted into the skin, the needle portions dissolve and administer the inactivated Japanese encephalitis virus into the skin (hereinafter, this microneedle array may also be referred to as an "autolytic microneedle array").

In a second embodiment, the needle portions have an inactivated Japanese encephalitis virus in a form in which the inactivated Japanese encephalitis virus is coated or deposited on the surfaces thereof, and when the needle portions are inserted into the skin, the needle portions do not dissolve in a living body, but the needle portions can administer the inactivated Japanese encephalitis virus present on the surfaces thereof into the skin (hereinafter this microneedle array may also be referred to as a "coating type microneedle array").

Preferably, the microneedle array is an autolytic microneedle array, and the needle portions contain an inactivated Japanese encephalitis virus.

### [Autolytic microneedle array]

The height (length) of a needle portion indicates the length of a perpendicular line that is suspended from the tip of the needle portion to a frustum portion or a sheet portion (in a case where a frustum portion is not present). The height (length) of a needle portion is not particularly limited, and it is preferably 50 µm or more and 3000 µm or less, more preferably 100 µm or more and 1500 µm or less, and further preferably 100 µm or more and 1000 µm or less. If the length of a needle portion is 50 µm or more, the inactivated Japanese encephalitis virus can be administered via transdermal administration. On the other hand, by setting the length of a needle portion to be 3000 µm or less, generation of pain caused by the contact of the needle portion with a nerve can be preferably prevented, and bleeding can be preferably avoided.

In a single microneedle array, 1 to 2000 needle portions are preferably arranged, 3 to 1000 needle portions are more preferably arranged, and 5 to 500 needle portions are further preferably arranged. When a single microneedle array contains two needle portions, the interval between the needle portions is indicated with a distance between the feet of perpendicular lines that are suspended from the tips of the needle portions to frustum portions or sheet portions (in a case where frustum portions are not present). When a single microneedle array contains three or more needle portions, the interval among the arranged needle portions is indicated with a mean value of the distances between the feet of perpendicular lines that are suspended from the tips of all needle portions to the frustum portions or sheet portions (in a case where frustum portions are not present) of the closest needle portions. The interval of the needle portions is preferably 0.1 mm or more and 10 mm or less, more preferably 0.2 mm or more and 5 mm or less, and further preferably 0.3 mm or more and 3 mm or less.

Each needle portion contains an inactivated Japanese encephalitis virus. The needle portion preferably further contains at least one type of a water-soluble polymer and a disaccharide, and more preferably further contains at least one type of an electrically neutral water-soluble polymer and a disaccharide. The needle portion contains a surfactant (preferably, an electrically neutral surfactant), as necessary.

The needle portion contains an inactivated Japanese encephalitis virus. For example, a formalin-inactivated virus preparation obtained by preparing Japanese encephalitis virus from a culture solution of Vero cells (African green monkey kidney-derived cell line), a formalin-inactivated virus preparation prepared from mouse brain infected with Japanese encephalitis virus, and the like can be used.

The content of the inactivated Japanese encephalitis virus in all of the needle portions is not particularly limited. The content of the inactivated Japanese encephalitis virus is preferably 0.0001% to 10% by mass, more preferably 0.0001% to 1% by mass, and particularly preferably 0.0001% to 0.5% by mass, with respect to 100% by mass of the solid content of the needle portions.

The content of an inactivated Japanese encephalitis virus in all of the needle portions may be determined by adjusting the content of the inactivated Japanese encephalitis virus in a single microneedle array so that it is adapted to the dosage and administration of the present invention. The content of the inactivated Japanese encephalitis virus in a single microneedle array is not particularly limited, and the content of the inactivated Japanese encephalitis virus as a protein content is preferably 0.01 to 2.5 µg, and more preferably 0.01 to 2 µg.

The needle portion preferably contains a surfactant (preferably, an electrically neutral surfactant, and more preferably, a nonionic surfactant). Examples of the surfactant may include sugar alcohol fatty acid ester such as sucrose fatty acid ester, sorbitan fatty acid ester, glycerin fatty acid ester, polyglycerin fatty acid ester, propylene glycol fatty acid ester, polyoxyethylene sorbitan fatty acid ester, polyoxyethylene glycerin fatty acid ester, polyethylene glycol fatty acid ester, polyoxyethylene/polyoxypropylene copolymerized polymer, polyoxyethylene castor oil, polyoxyethylene hydrogenated castor oil, and octylphenol ethoxylate. Among the above-described surfactants, sorbitan fatty acid ester, polyoxyethylene/polyoxypropylene copolymerized polymer, or polyoxyethylene hydrogenated castor oil is particularly preferable. As such surfactants, commercially available products such as Tween (registered trademark) 80, Pluronic (registered trademark) F-68, HCO-60, and Triton (registered trademark) -X can also be used.

The additive amount of the surfactant is not particularly limited, and it is preferably 0.01 µg or more, and more preferably 0.1 µg or more, per microneedle array (area: 1 cm²).

The water-soluble polymer contained in the needle portion is not particularly limited. Examples of the water-soluble polymer may include polysaccharides, polyvinyl pyrrolidone, polyoxyethylene polyoxypropylene glycol, polyethylene glycol, and polyvinyl alcohol. Examples of the above-mentioned polysaccharides may include pullulan, dextran, dextrin, cellulose derivatives (for example, water-soluble cellulose derivatives obtained by partially modifying cellulose, such as hydroxypropyl cellulose and hydroxypropyl methyl cellulose), hydroxyethyl starch, and gum Arabic. The above-described components may be used alone as a single type, or may also be used as a mixture of two or more types. In order not to give damage to a human body even if the needle portion remains in the skin, the water-soluble polymer is preferably a substance that can dissolve in a living body (i.e. a bio-soluble substance).

Among the above-described substances, the water-soluble polymer contained in the needle portion is preferably at least one type selected from the group consisting of hydroxyethyl starch, dextran, polyvinyl pyrrolidone, polyoxyethylene polyoxypropylene glycol, polyethylene glycol, and polyvinyl alcohol; and is particularly preferably hydroxyethyl starch. Further, in order to inhibit aggregation when the water-soluble polymer is mixed with the inactivated Japanese encephalitis virus, uncharged polysaccharides are more preferable. The water-soluble polymer contained in the needle portion may be the same as or different from the water-soluble polymer contained in the sheet portion.

The weight (mass) average molecular weight of the water-soluble polymer contained in the needle portion is preferably 5,000 or more and 100,000 or less, more preferably 10,000 or more and 100,000 or less, and further preferably 30,000 or more and 90,000 or less.

The needle portion (in particular, the tip region of the needle portion) may contain disaccharides. Examples of the disaccharides may include sucrose, lactulose, lactose, maltose, trehalose, and cellobiose. Of these, sucrose is particularly preferable.

The water-soluble polymer or the disaccharide accounts for preferably 50% by mass or more of the total solid content of the needle portion. The water-soluble polymer or the disaccharide accounts for more preferably 55% by mass or more of the total solid content of the needle portion, further preferably 60% by mass or more of the total solid content of the needle portion, and particularly preferably 65% by mass or more of the total solid content of the needle portion.

The upper limit is not particularly limited, but the water-soluble polymer or the disaccharide accounts for preferably 99% by mass or less of the total solid content of the needle portion, more preferably 95% by mass or less of the total solid content of the needle portion, and further preferably 90% by mass or less of the total solid content of the needle portion.

When the water-soluble polymer or the disaccharide accounts for 50% by mass or more of the total solid content of the needle portion, favorable puncturing properties and favorable medicinal effects can be achieved.

The ratio of the water-soluble polymer or the disaccharide to the total solid content of the needle portion can be measured by the following method, but the measurement method is not particularly limited thereto. As a measurement method, for example, the needle portion of the produced microneedle array is cut, and the needle portion is dissolved in a buffer solution (a buffer solution suitable for dissolving the water-soluble polymer constituting the needle portion, such as a phosphate buffered saline (PBS)), and the amount of the water-soluble polymer or the disaccharide in the solution can be measured by a high performance liquid chromatography method.

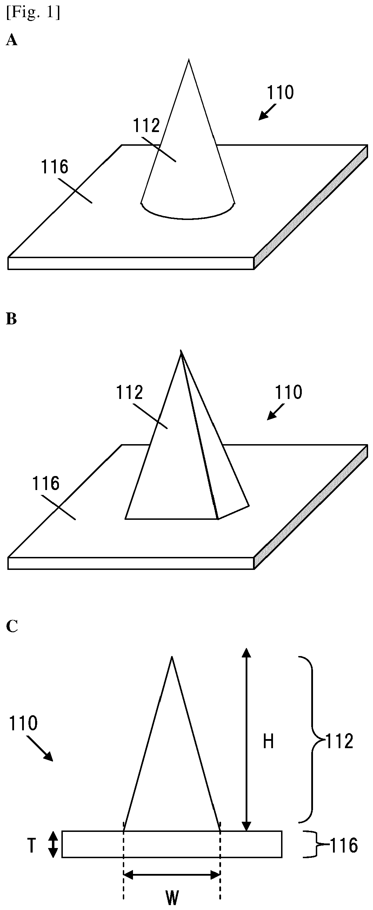

The sheet portion is a base for supporting the needle portions. The sheet portion has a planar shape, like a sheet portion 116 as shown in Fig. 1 to Fig. 8. The upper surface of the sheet portion indicates a surface on which a plurality of needle portions are arranged in an array state.

The area of the sheet portion is not particularly limited, and it is preferably 0.005 to 1000 mm², more preferably 0.05 to 500 mm², and further preferably 0.1 to 400 mm².

The thickness of the sheet portion is indicated with a distance between the surface contacted with the frustum portion or the needle portion and the surface on the opposite side. The thickness of the sheet portion is preferably 1 µm or more and 2000 µm or less, more preferably 3 µm or more and 1500 µm or less, and further preferably 5 µm or more and 1000 µm or less.

The sheet portion contains preferably at least one type of a water-soluble polymer and a disaccharide, and more preferably at least one type of an electrically neutral water-soluble polymer and a disaccharide. The sheet portion may also comprise other additives (for example, a charged water-soluble polymer, etc.). Besides, the sheet portion preferably does not contain an inactivated Japanese encephalitis virus.

The water-soluble polymer contained in the sheet portion is not particularly limited, and examples of the water-soluble polymer contained in the sheet portion may include polysaccharides, polyvinyl pyrrolidone, polyoxyethylene polyoxypropylene glycol, polyethylene glycol, and polyvinyl alcohol. Examples of the polysaccharides may include pullulan, dextran, dextrin, a cellulose derivative (for example, a water-soluble cellulose derivative obtained by partially modifying cellulose, such as hydroxypropyl cellulose and hydroxypropyl methyl cellulose), hydroxyethyl starch, and gum Arabic. The above-described components may be used alone as a single type, or may also be used as a mixture of two or more types.

Among the above-described substances, the water-soluble polymer contained in the sheet portion is preferably at least one type selected from the group consisting of hydroxyethyl starch, dextran, polyvinyl pyrrolidone, polyoxyethylene polyoxypropylene glycol, polyethylene glycol, and polyvinyl alcohol; and is particularly preferably hydroxyethyl starch.

The weight (mass) average molecular weight of the water-soluble polymer contained in the sheet portion is preferably 5,000 or more and 100,000 or less, more preferably 10,000 or more and 100,000 or less, and further preferably 30,000 or more and 90,000 or less.

The sheet portion may contain disaccharides. Examples of the disaccharides may include sucrose, lactulose, lactose, maltose, trehalose, and cellobiose. Of these, sucrose is particularly preferable.

Particularly preferably, there can be used a microneedle array, in which needle portions contain at least one type of a water-soluble polymer and a disaccharide, an inactivated Japanese encephalitis virus, and a surfactant; and a sheet portion contains at least one type of a water-soluble polymer and a disaccharide.

The microneedle array is composed of a plurality of needle portions arranged in an array state on the upper surface side of the sheet portion. The needle portion is a convex structure having a tip, and is not limited to a needle shape having a sharp tip, and the tip may have a blunt shape.

The shape of the needle portion is not particularly limited, and examples thereof may include a conical shape, a polygonal pyramid shape (a quadrangular pyramid shape, etc.), and a spindle shape. For example, the needle portion has a shape, like a needle portion 112 as shown in Fig. 1 to Fig. 8. The entire shape of the needle portion may be a conical or polygonal pyramid (a quadrangular pyramid, etc.), or the needle portion may have a structure, in which the inclination (angle) of the side surface of the needle portion is continuously changed. Otherwise, it is also possible for the needle portion to adopt a multilayer structure having two or more layers, in which the inclination (angle) of the side surface of the needle portion changes discontinuously.

When the microneedle array is applied to the skin, it is preferable that the needle portion is inserted into the skin, so that the upper surface of the needle portion, or a part thereof, is allowed to come into contact with the skin.

When the microneedle array has a plurality of frustum portions between the sheet portion and the plurality of needle portions, the needle portions are arranged on the upper surfaces of the frustum portions arranged on the upper surface of the sheet portion. In this embodiment, it is preferable that the tip region of the needle portion contains at least one type of a water-soluble polymer and a disaccharide, and an inactivated Japanese encephalitis virus, and that the frustum portion and the sheet portion contain at least one type of a water-soluble polymer and a disaccharide. The water-soluble polymer contained in the needle portion may be the same as or different from the water-soluble polymer contained in the sheet portion.

The interface between the frustum portion (provided that it is the needle portion, if the frustum portion is not present) and the sheet portion is referred to as a basement. The distance between the farthest points in the basement of a single needle portion is preferably 50 µm or more and 2000 µm or less, more preferably 100 µm or more and 1500 µm or less, and further preferably 200 µm or more and 1000 µm or less.

### [Coating type microneedle array]

In the coating type microneedle array, the height (length) of a needle portion, the number of needle portions, the intervals of needle portions, the content of an inactivated Japanese encephalitis virus in all of the needle portions, the content of an inactivated Japanese encephalitis virus in a single microneedle array, the area of a sheet portion, the thickness of a sheet portion, the shape of a needle portion, the presence or absence of a frustum portion(s), and the like can be determined as in the case of the autolytic microneedle array.

As materials for the needle portions and the sheet portion of the coating type microneedle array, the same materials as those for the autolytic microneedle array can be used. However, the materials are preferably composed of at least one of organic materials and resin materials. Examples of the inorganic materials may include: metals, such as silicon, silicon dioxide, ceramics, stainless steel, iron, aluminum, titanium, nickel, molybdenum, chromium, cobalt, copper, lead, niobium, tantalum, zirconium, tin, gold, and silver; and their alloys thereof. Examples of the resin materials may include: biodegradable polymers, such as polylactic acid, polyglycolide, polylactic acid-co-polyglycolide, pullulan, capronolactone, polyurethane, and polyanhydride; and non-degradable polymers, such as polycarbonate, polymethacrylic acid, ethylene vinyl acetate, polytetrafluoroethylene, acrylonitrile-butadiene-styrene copolymer, cycloolefin polymer (COP), and cycloolefin copolymer (COC).

A surface treatment may be performed on the surface of the needle portion to make it easier to get wet, so that the inactivated Japanese encephalitis virus can be thinly applied onto the surface of the needle portion. Examples of the treatment of increasing wettability may include a treatment of roughening the surface and a plasma treatment.

### [Preferred embodiments of microneedle array]

Hereafter, preferred embodiments of the present invention will be described in accordance with drawing attached herewith. However, the present invention is not limited to such embodiments.

Fig. 1 to Fig. 8 show a microneedle 110 that is a partially enlarged view of a microneedle array. A microneedle array is configured by forming a plurality of needle portions 112 on a sheet portion 116 (wherein, in each figure, only one needle portion 112, or one frustum portion 113 and one needle portion 112 are shown on a sheet portion 116, and this is referred to as a microneedle 110). The needle portion 112 is formed, such that the needle portion 112 becomes thinner from the surface of the sheet portion 116 to the top of the needle portion 112. The shape of the needle portion 112 is not limited, as long as it protrudes such that it entirely becomes thinner to the top direction. The needle portion 112 may be formed, for example, with another pyramid having a different number of angles, or with a cone.

In Fig. 1A, the needle portion 112 has a conical shape, and in Fig. 1B, the needle portion 112 has a quadrangular pyramid shape. In Fig. 1C, "H" indicates the height of the needle portion 112, "W" indicates the diameter (width) of the needle portion 112, and "T" indicates the height (thickness) of the sheet portion 116.

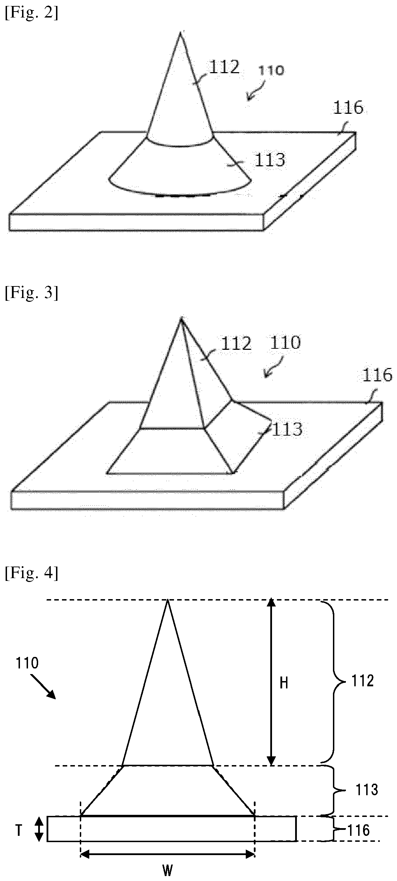

Fig. 2 and Fig. 3 show a microneedle 110 having another shape, in which a frustum portion 113 and a needle portion 112 are formed on the surface of a sheet portion 116. In Fig. 2, the frustum portion 113 has the shape of a truncated cone, and the needle portion 112 has the shape of a cone. Further, in Fig. 3, the frustum portion 113 has the shape of a truncated quadrangular pyramid, and the needle portion 112 has the shape of a quadrangular pyramid. However, the shape of the needle portion is not limited to these shapes.

Fig. 4 is a cross-sectional view of the microneedle 110 shown in Figs. 2 and 3. In Fig. 4, "H" indicates the height of the needle portion 112, "W" indicates the diameter (width) of the basement, and "T" indicates the height (thickness) of the sheet portion 116.

The microneedle array preferably has the shape of the microneedle 110 shown in Fig. 4, rather than the shape of the microneedle 110 shown in Fig. 1C. By adopting such a structure, the volume of the entire needle portion becomes large, and a larger number of inactivated Japanese encephalitis virus can be concentrated on the upper end of the needle portion during the production of the microneedle array.

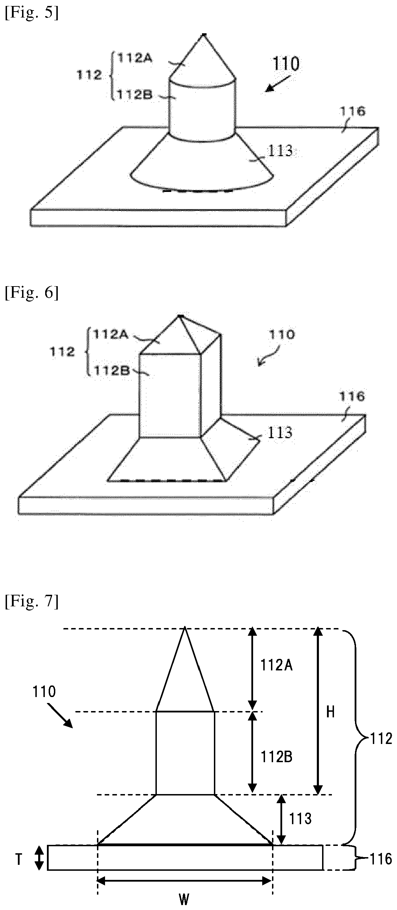

Fig. 5 and Fig. 6 show a microneedle 110 having a further different shape.

A needle portion first layer 112A shown in Fig. 5 has a conical shape, and a needle portion second layer 112B has a columnar shape. A needle portion first layer 112A shown in Fig. 6 has a quadrangular pyramid shape, and a needle portion second layer 112B has a quadrangular columnar shape. However, the shape of the needle portion is not limited to these shapes.

The shape of the tip of the top of the needle portion 112 is not particularly limited, as long as it can puncture the skin and provide a through hole in the stratum corneum. The top 21 of the needle portion 112 does not have to be a perfect apex and may have a curved or flat surface that can puncture the skin.

Fig. 7 is a cross-sectional view of the microneedle 110 shown in Figs. 5 and 6. In Fig. 7, "H" indicates the height of the needle portion 112, "W" indicates the diameter (width) of the basement, and "T" indicates the height (thickness) of the sheet portion 116.

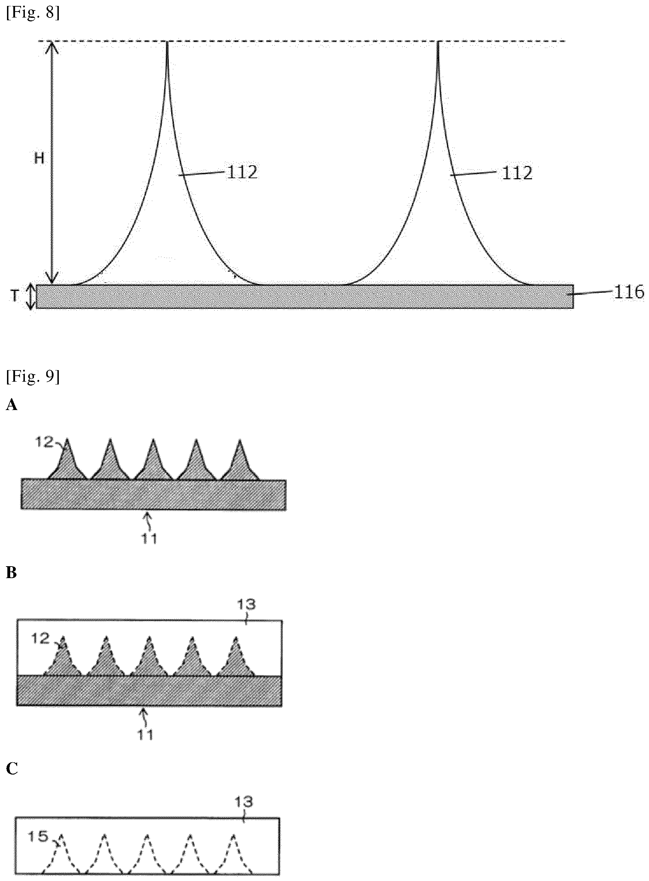

Fig. 8 is a cross-sectional view of another shape of microneedle, in which the inclination (angle) of the side surface of the needle portion 112 is continuously changed. In Fig. 8, "H" indicates the height of the needle portion 112, and "T" indicates the height (thickness) of the sheet portion 116.

In the microneedle array, the needle portions are preferably arranged at intervals of about 0.1 to 10 microneedles per 1 mm in a row. The microneedle array more preferably has 1 to 10,000 microneedles per 1 cm². By setting the density of the microneedles to be 1 microneedle/cm² or more, the skin can be perforated efficiently. On the other hand, and by setting the density of the microneedles to 10000 microneedles/cm² or less, the microneedle array is able to sufficiently puncture the skin. The density of the needle portions is preferably 10 to 5000 microneedles/cm², more preferably 25 to 1000 microneedles/cm², and particularly preferably 25 to 400 microneedles/cm².

The microneedle array can be supplied in a sealed storage form together with a desiccant. As such a desiccant, a known desiccant (for example, silica gel, quicklime, calcium chloride, silica alumina, a sheet-like desiccant, etc.) can be used.

### [Method for producing autolytic microneedle array]

An autolytic microneedle array can be produced by the following method, for example, in accordance with the method described in Japanese Patent Publication (Kokai) No. 2013-153866 A or International Publication WO2014/077242.

The method for producing an autolytic microneedle array is not particularly limited. The autolytic microneedle array is preferably obtained by a production method comprising (1) a step of producing a mold, (2) a step of preparing a solution containing at least one type of a water-soluble polymer and a disaccharide, and an inactivated Japanese encephalitis virus, (3) a step of filling the solution obtained in the step (2) into the mold to form a needle portion tip region, (4) a step of filling a solution containing at least one type of a water-soluble polymer and a disaccharide into the mold to form a rest of a needle portion, (as desired, a frustum portion), and a sheet portion, and (5) a step of peeling an array from the mold.

### (Production of mold)

Figs. 9A to 9C are process charts showing production of a mold. As shown in Fig. 9A, an original plate for producing a mold is first produced. There are two methods for producing this original plate 11.

The first method comprises applying a photoresist onto a Si substrate, and then performing exposure and development. Then, by performing etching according to RIE (reactive ion etching) or the like, an array comprising conical shaped portions (convex portions) 12 is produced on the surface of the original plate 11. Besides, when etching is performed according to RIE or the like to form conical shaped portions on the surface of the original plate 11, such conical shapes can be formed by etching from the oblique direction, while rotating the Si substrate. The second method is a method comprising processing a metal substrate such as Ni with a cutting tool such as a diamond bite, so as to form an array comprising shaped portions 12, such as, for example, quadrangular pyramids, on the surface of the original plate 11.

Next, a mold is produced. Specifically, as shown in Fig. 9B, a mold 13 is produced from the original plate 11. The following four methods can be applied as methods for producing a mold.

The first method is a method comprising pouring a silicone resin prepared by adding a hardener to PDMS (polydimethylsiloxane, for example, SYLGARD 184 (registered trademark) manufactured by Dow Corning) into the original plate 11, then performing a heat treatment at 100°C to harden it, and then peeling the obtained mold from the original plate 11. The second method is a method comprising pouring a UV (Ultraviolet) cured resin that is hardened by irradiation with ultraviolet rays into the original plate 11, then applying ultraviolet rays to the UV cured resin in a nitrogen atmosphere, and then peeling the obtained mold from the original plate 11. The third method is a method comprising pouring a solution obtained by dissolving a plastic resin such as polystyrene or PMMA (polymethylmethacrylate) in an organic solvent into the original plate 11 coated with a release agent, then drying it to volatilize the organic solvent and harden the residue, and then peeling the obtained mold from the original plate 11. The fourth method is a method of producing an inverted product by Ni electroforming.

Thereby, a mold 13, in which needle-shaped recessed portions 15 that are the inverted shapes of the cones or the pyramids of the original plate 11 are arranged in a two-dimensional array, is produced. The thus produced mold 13 is shown in Fig. 9C.

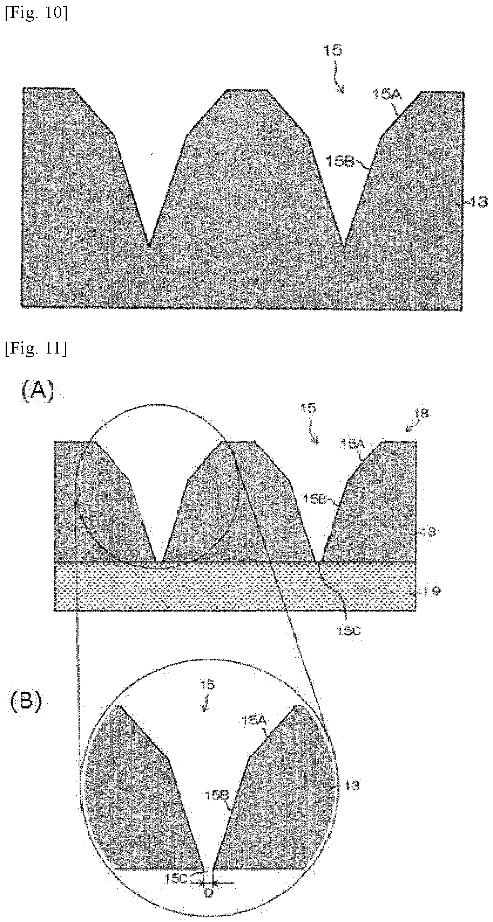

Fig. 10 shows another preferred embodiment of the mold 13. The needle-shaped recessed portion 15 comprises a tapered inlet portion 15A that narrows in the depth direction from the surface of the mold 13, and a tip recessed portion 15B that tapers in the depth direction. By forming the inlet portion 15A as a tapered shape, it becomes easy to fill the solution into the needle-shaped recessed portion 15.

Fig. 11 shows an embodiment of a mold complex 18 that is more preferable for production of a microneedle array. In Fig. 11, Fig. 11(A) shows the mold complex 18. In Fig. 11, Fig. 11(B) is an enlarged view of the portion surrounded with the circle in Fig. 11(A).

As shown in Fig. 11(A), the mold complex 18 comprises a mold 13, in which an air vent hole 15C is formed at the tip (bottom) of the needle-shaped recessed portion 15, and a gas permeation sheet 19 made of a material through which a gas is permeable but a liquid is impermeable, wherein the gas permeation sheet 19 is adhered to the back surface of the mold 13. The air vent hole 15C is formed as a through hole penetrating the back surface of the mold 13. Herein, the back surface of the mold 13 refers to the surface on the side where the air vent hole 15C is formed. Thereby, the tip of the needle-shaped recessed portion 15 communicates with the atmosphere through the air vent hole 15C and the gas permeation sheet 19.

By using such a mold complex 18, the solution to be filled into the needle-shaped recessed portion 15 does not permeate, and only the air existing in the needle-shaped recessed portion 15 can be removed from the needle-shaped recessed portion 15. Thereby, the transcribing ability for transcribing the shape of the needle-shaped recessed portion 15 onto a polymer is improved, and thus, a sharper needle portion can be formed.

The diameter D of the air vent hole 15C is preferably in the range of 1 to 50 µm. If the diameter D of the air vent hole 15C is less than 1 µm, the air vent hole 15C cannot sufficiently play a role as an air vent hole. On the other hand, if the diameter D of the air vent hole 15C exceeds 50 µm, the sharpness of the tip portion of the molded microneedle is impaired.

As a gas permeable sheet 19 made of a material that allows a gas to permeate but does not allow a liquid to permeate, for example, a gas permeable film (Sumitomo Electric Industries, Ltd., Poreflon (registered trademark), FP-010) can be preferably used.

As a material used for the mold 13, an elastic material or a metal material can be used. Of these, an elastic material is preferable, and a material having high gas permeability is more preferable. Oxygen permeability, which is representative gas permeability, is preferably 1 × 10⁻¹² (mL/s·m²·Pa) or more, and more preferably 1 × 10⁻¹⁰ (mL/s·m²·Pa) or more. It is to be noted that 1 mL is 10⁻⁶ m³. By setting the gas permeability within the above-described range, the air existing in the recessed portion of the mold 13 can be discharged from the mold side, and thus, a microneedle array having few defects can be produced. Specific examples of such a material may include silicone resins (for example, SYLGARD 184 manufactured by Dow Corning (registered trademark), and KE-1310ST (product number) manufactured by Shin-Etsu Chemical Co., Ltd.), ultraviolet curable resins, and plastic resins (for example, polystyrene and PMMA (polymethylmethacrylate)), all of which are melted or dissolved in a solvent. Among these resins, silicone rubber-based materials are preferable because they are durable against transcription by repeated pressurization and have good peelability from the materials. In addition, examples of the metal materials may include Ni, Cu, Cr, Mo, W, Ir, Tr, Fe, Co, MgO, Ti, Zr, Hf, V, Nb, Ta, α-aluminum oxide, zirconium oxide, stainless steel (for example, Stavax material (STAVAX) (trademark) of Bohler-Uddeholm K.K.), and alloys thereof. As a material for a frame 14, the same material as that for the mold 13 can be used.

### (Solution)

In the present invention, the following solutions are preferably prepared:
(i) a solution containing at least one type of a water-soluble polymer and a disaccharide, an inactivated Japanese encephalitis virus, and a surfactant, wherein the solution is for use in forming a needle portion tip region that is a part of a needle portion; and
(ii) a solution containing at least one type of a water-soluble polymer and a disaccharide, wherein the solution is for use in forming a needle portion root region other than the needle portion tip region in the needle portion, and a sheet portion (or a needle portion root region other than the needle portion tip region in the needle portion, a frustum portion, and a sheet portion).

The types of the water-soluble polymer, the disaccharide, and the surfactant are as described above herein.

The concentration of the water-soluble polymer or disaccharide in the above-described solution is different depending on the type of the substance used. In general, it is preferably 1% to 50% by mass. Further, the solvent used for dissolution may be a solvent having volatility, as well as water. Methyl ethyl ketone (MEK), alcohol, and the like can be used.

### (Formation of needle portion tip region)

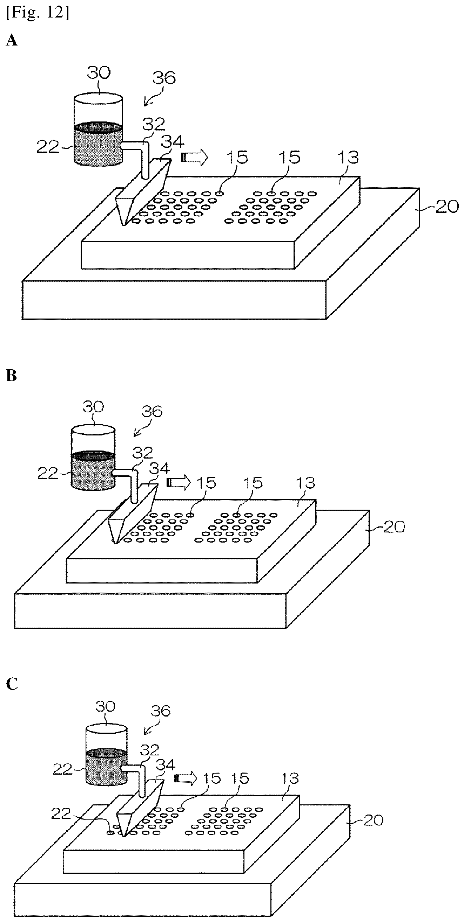

As shown in Fig. 12A, the mold 13 having two-dimensionally arranged needle-shaped recessed portions 15 is arranged on a base 20. On the mold 13, two sets of a plurality of needle-shaped recessed portions 15 are each arranged in a two-dimensional array of 5 x 5. A liquid-supplying device 36 having a tank 30 storing a solution 22 containing an inactivated Japanese encephalitis virus, a pipe 32 connected with the tank, and a nozzle 34 connected with the tip of the pipe 32 is prepared. Besides, in the present example, a case where the needle-shaped recessed portions 15 are two-dimensionally arranged in 5 x 5 is illustrated. However, the number of needle-shaped recessed portions 15 is not limited to 5 x 5, and the needle-shaped recessed portions 15 may be two-dimensionally arranged in M x N (wherein M and N each independently represent any given integer of 1 or more, preferably 2 to 30, more preferably 3 to 25, further preferably 3 to 20).

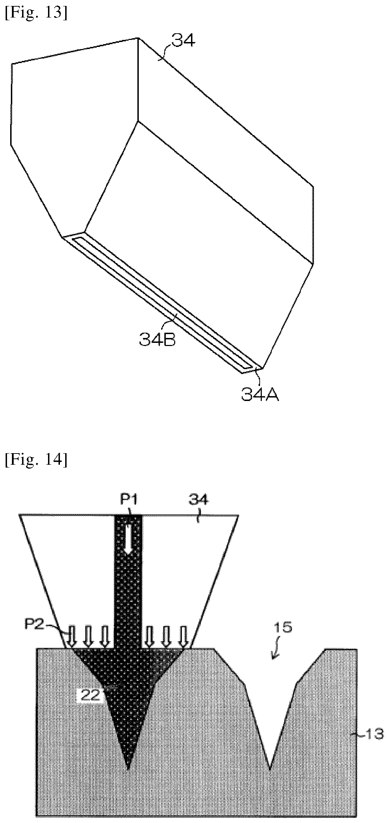

Fig. 13 shows a schematic perspective view of the tip portion of a nozzle. As shown in Fig. 13, the tip of a nozzle 34 comprises a flat surface lip portion 34A and a slit-shaped opening portion 34B. The slit-shaped opening portion 34B makes it possible, for example, to simultaneously fill a solution 22 containing an inactivated Japanese encephalitis virus into a plurality of needle-shaped recessed portions 15 that constitutes one row. The size (length and width) of the opening portion 34B is appropriately selected, depending on the number of needle-shaped recessed portions 15 to be filled at once. By increasing the length of the opening portion 34B, the solution 22 containing an inactivated Japanese encephalitis virus can be filled into a larger number of needle-shaped recessed portions 15 at once. Thereby, it becomes possible to improve productivity.

As a material used for the nozzle 34, an elastic material or a metal material can be used. For example, Teflon (registered trademark), stainless steel (SUS (Steel Use Stainless)), titanium and the like can be used.

As shown in Fig. 12B, the opening portion 34B of the nozzle 34 is adjusted to be positioned above the needle-shaped recessed portions 15. The lip portion 34A of the nozzle 34 and the surface of the mold 13 are in contact with each other. The solution 22 containing an inactivated Japanese encephalitis virus is supplied from the liquid-supplying device 36 to the mold 13, and the solution 22 containing an inactivated Japanese encephalitis virus is filled into the needle-shaped recessed portions 15 from the opening portion 34B of the nozzle 34. In the present embodiment, the plurality of needle-shaped recessed portions 15 constituting one row are simultaneously filled with the solution 22 containing an inactivated Japanese encephalitis virus. However, the present invention is not limited to this embodiment, and the needle-shaped recessed portions 15 can also be filled one by one.

When the mold 13 is made of a gas-permeable material, the solution 22 containing an inactivated Japanese encephalitis virus can be aspirated by sucking it from the back surface of the mold 13, and thus, the filling of the solution 22 containing an inactivated Japanese encephalitis virus into the needle-shaped recessed portions 15 can be promoted.

With reference to Fig. 12B, after the filling step, while the lip portion 34A of the nozzle 34 is allowed to come into contact with the surface of the mold 13, the liquid-supplying device 36 is allowed to relatively move to the direction perpendicular to the length direction of the opening portion 34B, as shown in Fig. 12C, so that the nozzle 34 is allowed to move to the needle-shaped recessed portions 15 which are not filled with the solution 22 containing an inactivated Japanese encephalitis virus. The opening portion 34B of the nozzle 34 is adjusted to be positioned above the needle-shaped recessed portions 15. In the present embodiment, although the example of moving the nozzle 34 has been described, the mold 13 may also be moved.

Since the liquid-supplying device moves, while the lip portion 34A of the nozzle 34 is allowed to come into contact with the surface of the mold 13, the nozzle 34 can scrape off the solution 22 containing an inactivated Japanese encephalitis virus that remains on the surface of the mold 13 other than the needle-shaped recessed portions 15. As a result, the solution 22 containing an inactivated Japanese encephalitis virus can be prevented from remaining in the mold 13 other than the needle-shaped recessed portions 15.

In order to reduce damage to the mold 13 and to suppress deformation due to compression of the mold 13 as much as possible, the pressing pressure of the nozzle 34 onto the mold 13, when moving, is preferably as small as possible. Moreover, in order to prevent the solution 22 containing an inactivated Japanese encephalitis virus from remaining on the sites of the mold 13, other than the needle-shaped recessed portions 15, it is desirable that at least one of the mold 13 or the nozzle 34 is a flexible, elastically deformable material.

By repeating the filling step of Fig. 12B and the moving step of Fig. 12C, the solution 22 containing an inactivated Japanese encephalitis virus is filled into the needle-shaped recessed portions 15 arranged in two dimensions of 5 x 5. When the solution 22 containing an inactivated Japanese encephalitis virus is filled into the two-dimensionally arranged 5 x 5 needle-shaped recessed portions 15, the liquid-supplying device 36 moves to adjacent two-dimensionally arranged 5 x 5 needle-shaped recessed portions 15. Then, the filling step of Fig. 12B and the moving step of Fig. 12C are repeated. Thus, the adjacent two-dimensionally arranged 5 x 5 needle-shaped recessed portions 15 are also filled with the solution 22 containing an inactivated Japanese encephalitis virus.

Regarding the above-mentioned filling step and moving step, it may be adequate to adopt (1) an embodiment, in which the solution 22 containing an inactivated Japanese encephalitis virus is filled into the needle-shaped recessed portions 15, while moving the nozzle 34, or (2) an embodiment, in which the nozzle 34 is temporarily stopped on the needle-shaped recessed portions 15, while moving the nozzle 34, and the solution 22 containing an inactivated Japanese encephalitis virus is filled into the needle-shaped recessed portions 15, and after the filling, the nozzle 34 is allowed to move again. Between the filling step and the moving step, the lip portion 34A of the nozzle 34 is allowed to come into contact with the surface of the mold 13.

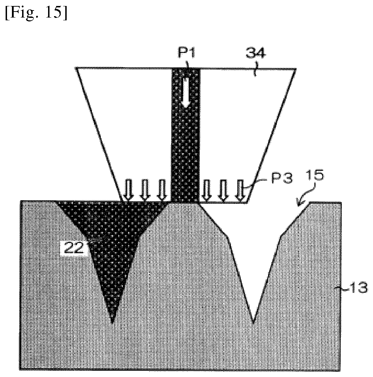

Fig. 14 is a partially enlarged view of the tip of the nozzle 34 and the mold 13, while the solution 22 containing an inactivated Japanese encephalitis virus is being filled into the needle-shaped recessed portion 15. As shown in Fig. 15, by applying a pressurizing force P1 into the nozzle 34, the filling of the solution 22 containing an inactivated Japanese encephalitis virus into the needle-shaped recessed portion 15 can be promoted. Further, when the solution 22 containing an inactivated Japanese encephalitis virus is filled into the needle-shaped recessed portion 15, a pressurizing force P2 for allowing the nozzle 34 to come into contact with the surface of the mold 13 is preferably set to be equal to or greater than the pressurizing force P1 in the nozzle 34. By setting the pressurizing force P2 ≥ the pressurizing force P1, the solution 22 containing an inactivated Japanese encephalitis virus can be prevented from leaking from the needle-shaped recessed portion 15 to the surface of the mold 13.

Fig. 15 is a partially enlarged view of the tip of the nozzle 34 and the mold 13, while the nozzle 34 is moving. When the nozzle 34 is allowed to move relative to the mold 13, a pressurizing force P3 for allowing the nozzle 34 to come into contact with the surface of the mold 13 is preferably set to be smaller than the pressurizing force P2 that allows the nozzle 34 during filling to come into contact with the surface of the mold 13. This is to reduce damage to the mold 13 and to suppress deformation due to compression of the mold 13.

When the filling of a plurality of needle-shaped recessed portions 15 composed of 5 x 5 is completed, the nozzle 34 is allowed to move to a plurality of adjacent needle-shaped recessed portions 15 composed of 5 x 5. Regarding the liquid supply, when the nozzle is allowed to move to the plurality of adjacent needle-shaped recessed portions 15 composed of 5 x 5, the supply of the solution 22 containing an inactivated Japanese encephalitis virus is preferably terminated. There is a certain distance from the needle-shaped recessed portions 15 in the fifth row to the needle-shaped recessed portions 15 in the next first row. When the nozzle 34 is allowed to move over the distance, if the solution 22 containing an inactivated Japanese encephalitis virus is continuously supplied, the hydraulic pressure in the nozzle 34 may become too high in some cases. As a result, the solution 22 containing an inactivated Japanese encephalitis virus may flow out from the nozzle 34 to the sites of the mold 13, other than the needle-shaped recessed portions 15, in some cases. In order to prevent this phenomenon, the hydraulic pressure in the nozzle 34 is detected, and when the hydraulic pressure is judged to be too high, the supply of the solution 22 containing an inactivated Japanese encephalitis virus is preferably terminated.

Besides, in the above description, the method of supplying the solution containing the inactivated Japanese encephalitis virus, using a dispenser having a nozzle, has been explained. However, in addition to the application using the dispenser, application by bar coating, spin coating, coating with a spray, etc. can also be applied.

In the present invention, a drying treatment is preferably performed, after a solution containing an inactivated Japanese encephalitis virus has been supplied to a needle-shaped recessed portion.

Preferably, a microneedle array can be produced by: a step of drying a needle portion-forming mold filled with a solution containing an inactivated Japanese encephalitis virus, so as to form a part of a needle portion; and a step of filling a solution containing at least one type of a water-soluble polymer and a disaccharide into the upper surface of a part of the needle portion formed as described above, and then drying it.

The conditions for drying the needle-forming mold filled with the solution containing an inactivated Japanese encephalitis virus are preferably conditions under which the water content of the aforementioned solution reaches 20% or less at 30 to 300 minutes after initiation of the drying.

Particularly preferably, the above-mentioned drying can be controlled, so that a temperature or lower at which the inactivated Japanese encephalitis virus is not deactivated is maintained and the water content of the solution reaches 20% or less at 60 minutes or more after initiation of the drying.

As a method of controlling the drying rate described above, any given means capable of delaying drying, such as, for example, temperature, humidity, drying air volume, the use of a container, and the volume and/or shape of the container, can be applied.

Drying can be preferably carried out in a state in which the needle portion-forming mold filled with the solution containing an inactivated Japanese encephalitis virus is covered with a container, or is contained in a container.

The temperature applied during the drying is preferably 1°C to 45°C, and more preferably 1°C to 40°C.

The relative humidity applied during the drying is preferably 10% to 95%, more preferably 20% to 95%, and further preferably 30% to 95%.

### (Formation of sheet portion)

Several embodiments of a step of forming a sheet portion will be described.

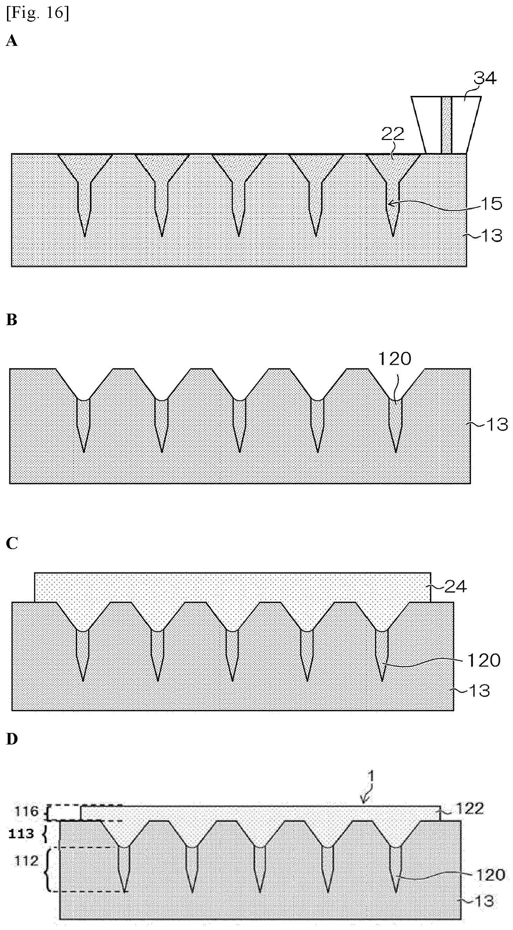

A first embodiment of the step of forming a sheet portion will be described with reference to Figs. 16A to 16D. A solution 22 containing an inactivated Japanese encephalitis virus is filled from a nozzle 34 into needle-shaped recessed portions 15 of a mold 13. Subsequently, as shown in Fig. 16B, the solution 22 containing an inactivated Japanese encephalitis virus is dried and solidified, so as to form layers 120 containing the inactivated Japanese encephalitis virus in the needle-shaped recessed portions 15. Subsequently, as shown in Fig. 16C, a solution 24 containing at least one of a water-soluble polymer and a disaccharide is coated using a dispenser onto the mold 13, on which the layers 120 containing the inactivated Japanese encephalitis virus have been formed. In addition to the coating using a dispenser, bar coating, spin coating, coating with a spray or the like, etc. can be applied. Since the layers 120 containing the inactivated Japanese encephalitis virus are solidified, the inactivated Japanese encephalitis virus can be prevented from spreading in the above-described solution 24. Subsequently, as shown in Fig. 17D, the solution 24 is dried and solidified, so as to form a microneedle array 1 composed of a plurality of needle portions 112, frustum portions 113, and a sheet portion 116.

In the first embodiment, in order to promote the filling of the solution 22 containing an inactivated Japanese encephalitis virus, and the solution 24 containing at least one type of a water-soluble polymer and a disaccharide, into the needle-shaped recessed portions 15, it is also preferable to carry out pressurization from the surface of the mold 13 and decompression suction from the back surface of the mold 13.

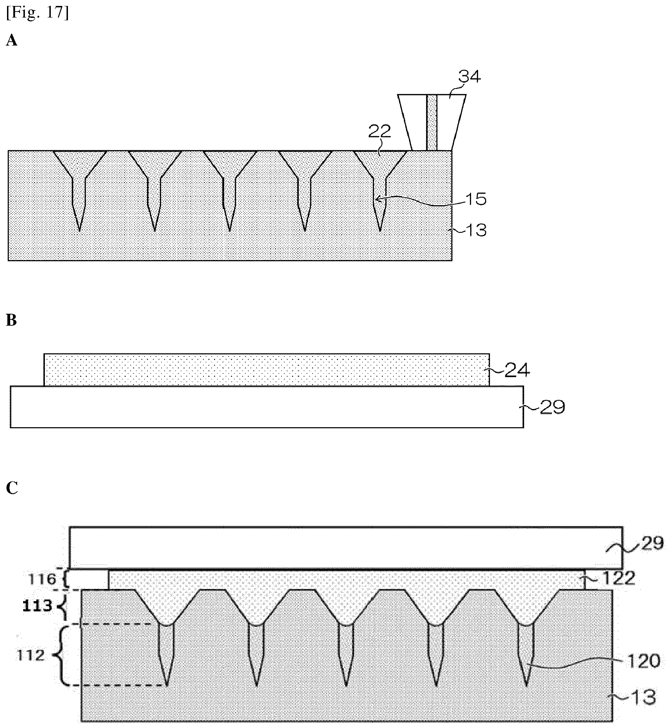

Next, a second embodiment will be described with reference to Figs. 17A to 17C. As shown in Fig. 17A, a solution 22 containing an inactivated Japanese encephalitis virus is filled from a nozzle 34 into needle-shaped recessed portions 15 of a mold 13. Subsequently, as with Fig. 16B, by drying and solidifying the solution 22 containing an inactivated Japanese encephalitis virus, layers 120 containing the inactivated Japanese encephalitis virus are formed in the needle-shaped recessed portions 15. Subsequently, as shown in Fig. 17B, a solution 24 containing at least one of a water-soluble polymer and a disaccharide is applied onto another support 29. Examples of the support 29 that can be used herein may include, but are not limited to, polyethylene, polyethylene terephthalate, polycarbonate, polypropylene, acrylic resin, triacetyl cellulose, and glass. Subsequently, as shown in Fig. 17C, the solution 24 formed on the support 29 is superposed on the mold 13, in which the layers 120 containing the inactivated Japanese encephalitis virus are formed in the needle-shaped recessed portions 15. Thereby, the above-described solution 24 is filled into the needle-shaped recessed portions 15. Since the layers containing the inactivated Japanese encephalitis virus are solidified, the inactivated Japanese encephalitis virus can be prevented from spreading in the above-described solution 24. Subsequently, the solution 24 is dried and solidified, so as to form a microneedle array composed of a plurality of needle portions 112, frustum portions 113, and a sheet portion 116.

In the second embodiment, in order to promote the filling of the solution 24 containing at least one type of a water-soluble polymer and a disaccharide into the needle-shaped recessed portions 15, it is also preferable to carry out pressurization from the surface of the mold 13 and decompression suction from the back surface of the mold 13.

The method of drying the solution 24 containing at least one type of a water-soluble polymer and a disaccharide may be a step of volatilizing the solvent in the solution. The drying method is not particularly limited, and for example, a drying method such as heating, blowing, or depressurization is used. The drying treatment can be carried out under conditions of 1°C to 50°C and 1 to 72 hours. In the case of blowing air, a method of blowing warm air at a rate of 0.1 to 10 m/sec can be applied. The drying temperature is preferably a temperature, at which an inactivated Japanese encephalitis virus contained in the solution 22 containing the inactivated Japanese encephalitis virus is not thermally deteriorated.

### (Peeling)

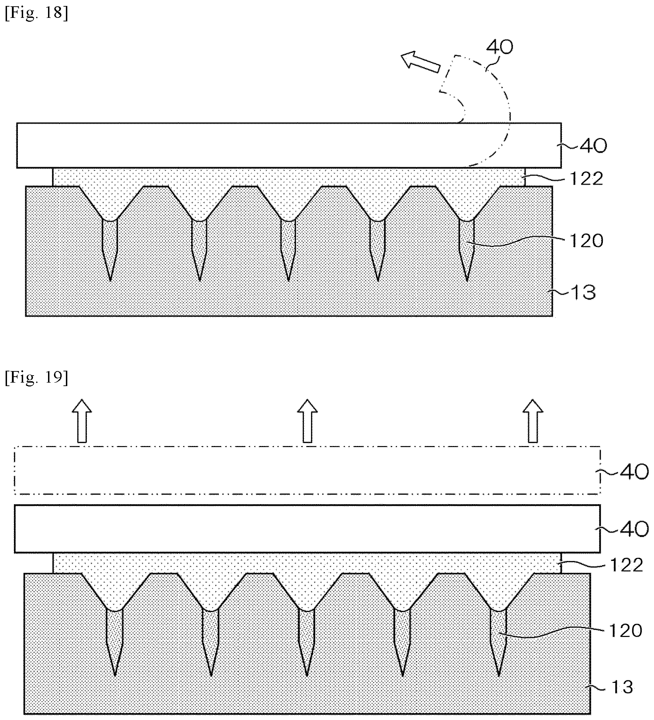

The method of peeling the microneedle array from the mold 13 is not particularly limited. It is preferable that the needle portions do not bend or break during peeling. Specifically, as shown in Fig. 18, a sheet-shaped base material 40, on which an adhesive layer having certain adhesiveness is formed, is attached onto the microneedle array, and then, the base material 40 can be peeled off from the end portion. However, by this method, the needle portions are likely to bend. Therefore, as shown in Fig. 19, a method of installing a suction cup (not shown in the figure) on the base material 40 on the microneedle array and then pulling it vertically, while sucking it with air, can be applied. The support 29 may be used as such a base material 40.

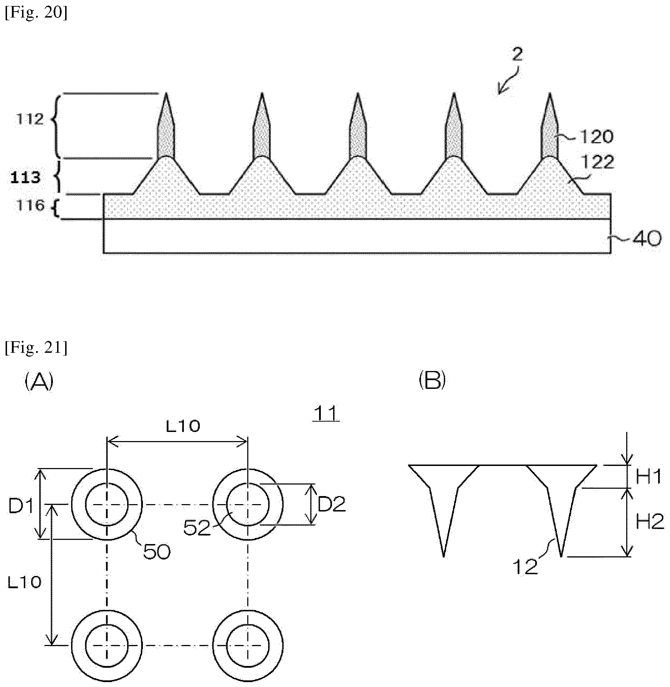

Fig. 20 shows a microneedle array 2 peeled from the mold 13. The microneedle array 2 is composed of a base material 40, and needle portions 112, frustum portions 113 and a sheet portion 116, which are formed on the base material 40. The needle portion 112 has a conical shape or a polygonal pyramid shape, at least, at the tip, but the needle portion 112 is not limited to this shape.

### [Method for producing coating type microneedle array]

The method for producing a coating type microneedle array is not particularly limited, as long as needle portions can be formed on a sheet portion according to the method. The coating type microneedle array is preferably obtained by a production method comprising (1) a step of producing a microneedle array, (2) a step of preparing a solution containing an inactivated Japanese encephalitis virus, and (3) a step of coating the solution containing an inactivated Japanese encephalitis virus onto the microneedle array.

As such a method for producing a coating type microneedle array, a method for producing an autolytic microneedle can also be applied. Specifically, a coating type microneedle array can be produced according to the method for producing an autolytic microneedle, from which (2) a step of preparing a solution containing at least one type of a water-soluble polymer and a disaccharide, and an inactivated Japanese encephalitis virus, and (3) a step of filling the solution obtained in the step (2) in a mold to form a needle portion tip region, are omitted. To the produced autolytic microneedle array, the solution containing an inactivated Japanese encephalitis virus may be applied.

As such a method for producing a coating type microneedle array, the coating type microneedle array can be produced by using various known techniques, depending on the materials. For example, molding techniques, such as injection molding, extrusion molding, imprinting, hot embossing, or casting, can be used to form a microneedle array. For example, an original plate of a microneedle array is formed, the concave and convex of the original plate are then inverted to form an intaglio, and a thermoplastic resin is then filled into the intaglio, so as to form a microneedle array composed of the thermoplastic resin. Otherwise, microneedles can also be formed by using a photolithography method, a modeling method using a 3D printer, a wet etching method, a dry etching method, a sandblasting method, a laser processing method, or a precision machining technique. Further, a plurality of molding techniques and precision machining techniques may be combined with one another to form a microneedle array.

In the step of preparing a solution containing an inactivated Japanese encephalitis virus, there can be used aqueous solvents including water and alcoholic solvents such as ethanol, isopropanol, diethylene glycol or glycerin, which are less harmful to human bodies even if such solvents remain in small amounts in the bodies. As other solvents, solvents including aliphatic hydrocarbons, aromatic hydrocarbons such as toluene, halogen hydrocarbons, esters such as ethyl acetate and propyl acetate, nitriles, and amides can be used alone as a single type, or in combination of two or more types. In order to apply or deposit a drug on the surface of the needle portion, the solution is preferably mixed with a high-molecular-weight compound and/or a low-molecular-weight compound. Examples of such compounds may include: cellulosic compounds such as polyethylene oxide, hydroxypropyl cellulose, and polyhydroxypropyl methyl cellulose; high-molecular-weight compounds such as dextran, polyethylene glycol, polyvinyl alcohol, polyvinyl pyrrolidone, pullulan and hyaluronic acid; and low-molecular-weight compounds, including salts such as sodium chloride, and sugars such as glucose. In particular, hydroxypropyl cellulose, dextran, polyethylene glycol, polyvinyl pyrrolidone, pullulan, hyaluronic acid, and the like are preferable.

In the step of coating the solution containing an inactivated Japanese encephalitis virus onto the microneedle array, an inkjet method of applying the solution to the needle portion by ejecting the solution via a nozzle, etc., a dispenser method of applying the solution to the needle portion via a nozzle, etc., an immersion method (dipping method) of immersing the needle portion in the solution so that the solution is attached to the needle portion, and the like can be used. Among these methods, the immersion method is effective for intensively applying the inactivated Japanese encephalitis virus to the tip region of the needle portion.

According to the immersion method, the top of the needle portion is directed downward, and the needle portion is then immersed in the solution. After that, the needle portion is pulled up from the solution, and the solvent of the solution is then evaporated, so as to form a layer containing the inactivated Japanese encephalitis virus on the surface of the needle portion. The amount of the inactivated Japanese encephalitis virus applied can be adjusted by regulating the depth of immersion of the needle portion, the immersion time, and the like.

### [Administration of microneedle array and intended use of microneedle array]

The present invention further relates to a method for preventing Japanese encephalitis, comprising administering to a subject, a microneedle array having a sheet portion and a plurality of needle portions present on the upper surface of the sheet portion, wherein the needle portions contain or carry an inactivated Japanese encephalitis virus (hereinafter referred to as a "microneedle array").

The present invention further relates to a method for imparting immunity against Japanese encephalitis to a subject, comprising administering to the subject, the microneedle array.

The subject, to which the microneedle array is administered, is not particularly limited, and it is preferably a mammal, and more preferably a human.

The preferred embodiments of the microneedle array and the preferred dose of the microneedle array are as described above in the present description.

The microneedle array can also be administered using an applicator. The applicator is not particularly limited, and those having embodiments described, for example, in Japanese Patent Publication (Kokai) No. 2018-191783 A, Japanese Patent Publication (Kokai) No. 2019-097885 A, etc. can be used.

The present invention further relates to the above-described microneedle array for use in a treatment of preventing Japanese encephalitis, and the above-described microneedle array for use in a treatment of imparting immunity against Japanese encephalitis to a subject.

The present invention further relates to use of the above-described microneedle array for the production of a Japanese encephalitis preventive agent, and use of the above-described microneedle array for the production of a Japanese encephalitis vaccine agent.

The preferred embodiments of the microneedle array are as described above in the present description.

Hereinafter, the present invention will be more specifically described in the following examples. It is to be noted that, in the following examples, the materials used, the amounts and ratios thereof, and the details of the treatment and the treatment process may be suitably modified, unless they overstep the spirit and the scope of the invention. Accordingly, the invention should not be restrictively interpreted by the specific examples mentioned below.

### Examples

The abbreviations used in the Examples have the following meanings. HES: Hydroxyethyl Starch 70000 (Fresenius Kabi Co., Ltd.) (weight average molecular weight: 70000)
DEX: Dextran 70000 (Meito Sangyo Co., Ltd.) (weight average molecular weight: 70000)
CS: Sodium Chondroitin Sulfate (Maruha Nichiro Corporation) (weight average molecular weight: 90000)
Suc: Purified white sugar (sucrose) (Fuji Film Wako Pure Chemical Industries, Ltd.)
GluNa: Sodium L-glutamate (Fujifilm Wako Pure Chemical Industries, Ltd.)
Tw: Tween 80 (Seppic)
EMEM: Eagle's minimum essential medium
FBS: Bovine fetal serum
CPE: Cytopathic effect
MNA-H: Microneedle Japanese encephalitis vaccine high dose
MNA-L: Microneedle Japanese encephalitis vaccine low dose
SC: Japanese encephalitis vaccine for subcutaneous injection
ICDRG: International Contact Dermatitis Research Group
IgG: Immunoglobulin G
BSA: Bovine serum albumin
PBS: Phosphate-buffered saline
DPBS: Dalbecco's phosphate-buffered saline
Tris: Trishydroxymethylaminomethane
HRP: Horseradish peroxidase
TMB: Tetramethylbenzidine
HIV: Human immunodeficiency virus
HBs: Hepatitis B virus surface antigen
HCV: Hepatitis C virus
TP: Treponema pallidum
HTLV-1: Human T-cell leukemia virus type 1

### < Production of microneedle array >

### (Production of mold)

On the surface of a smooth Ni plate with a side of 40 mm, 100 of acicular structure-shaped portions 12, in each of which a cone 52 having a diameter D2 of 300 µm and a height H2 of 500 µm is formed on a truncated cone 50 having a bottom surface of 500 µm (as a diameter D1) and a height H1 of 150 µm, were formed in the form of a square by grinding process at a pitch L1 of 1000 µm in a two-dimensional square arrangement, as shown in Fig. 21, thereby producing an original plate 11. On this original plate 11, a film of silicon rubber (SILASTIC MDX4-4210, manufactured by Dow Corning) was formed with a thickness of 0.6 mm, and thermosetting was performed on the film in a state in which the tip portion of the cone of the original plate 11 protruded 50 µm from the surface of the film, and then, peeling was performed. Thereby, an inverted product of the silicon rubber having a through hole with a diameter of about 30 µm was produced. This silicon rubber inverted product, in the center of which needle-shaped recessed portions were two-dimensionally arranged in an array of 10 columns x 10 rows, and from which the outside of a flat surface having a side of 30 mm was cut off, was used as a mold. The wider opening portion of the needle-shaped recessed portion was used as the front surface of the mold, and the surface having a through hole (air vent hole) with a diameter of 30 µm was used as the back surface of the mold.

### (Preparation of aqueous solution containing inactivated Japanese encephalitis virus (also referred to as "solution I"))

After concentration of the Japanese encephalitis vaccine stock solution (provided by General Incorporated Association, Research Institute for Microbial Diseases, Osaka University) by a centrifugal ultrafiltration method, the obtained concentrate was mixed with a water-soluble polymer, purified white sugar (sucrose) (Fuji Film Wako Pure Chemical Industries, Ltd.), sodium L-glutamate (Fujifilm Wako Pure Chemical Industries, Ltd.), and a surfactant. The amounts of individual components used are shown in Table 1 and Table 4. Besides, the amounts of individual components shown in Table 1 and Table 4 are the amounts of individual components per microneedle array. The area of a single microneedle array is 1 cm².

### (Preparation of solution that forms sheet portion (also referred to as "solution II"))

Hydroxyethyl Starch 70000 (HES, Freshenius Kabi Co., Ltd.) was dissolved in water to prepare an aqueous solution containing 56% by mass of HES. The amount used is shown in Table 1.

In addition, DEX and CS were dissolved in water at a ratio (mass ratio) of 7 : 3 to prepare an aqueous solution containing DEX and CS. The amount used is shown in Table 3.

Besides, the amounts of individual components shown in Table 1 and Table 3 are the amounts of individual components per microneedle array. The area of a single microneedle array is 1 cm².

### (Filling and drying of solution containing inactivated Japanese encephalitis virus)

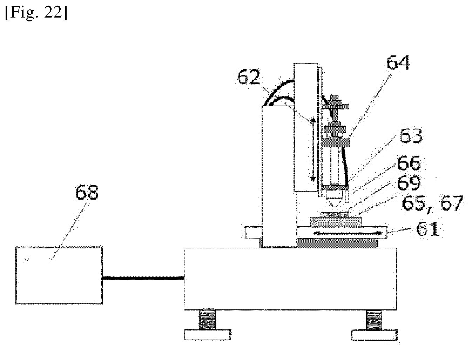

The filling device shown in Fig. 22 was used. The filling device comprises an X-axis driving portion 61 and a Z-axis driving portion 62 that control the relative position coordinates of a mold and a nozzle, a liquid-supplying device 64 (Ultramicro Determination Dispenser SMP-III, manufactured by Musashi Engineering Co., Ltd.) into which the nozzle 63 can be equipped, a suction table 65 for fixing a mold 69, a laser displacement meter 66 (HL-C201A, manufactured by Panasonic) for measuring the surface shape of the mold, a load cell 67 (LCX-A-500N, manufactured by Kyowa Electric Co., Ltd.) for measuring the nozzle pushing pressure, and a control mechanism 68 for controlling the Z-axis based on the measured value data of the surface shape and the pressing pressure.

A gas permeable film with a side of 15 mm (manufactured by Sumitomo Electric Industries, Ltd., Poreflon (registered trademark), FP-010) was placed on the horizontal suction table, and the mold was then placed on it, so that the surface thereof was facing up. The gas permeable film and the mold were fixed on the vacuum table by reducing the pressure from the back surface direction of the mold with a gauge pressure of 90 kPa.

A SUS (stainless steel)-made nozzle having such a shape as shown in Fig. 13 was prepared, and a slit-shaped opening portion having a length of 12 mm and a width of 0.2 mm was formed in the center of a lip portion thereof having a length of 20 mm and a width of 2 mm. This nozzle was connected with the liquid-supplying device. A solution containing an inactivated Japanese encephalitis virus (3 mL) was filled into the liquid-supplying device and the inside of the nozzle. The nozzle was adjusted, so that the opening portion could be located parallel to the first row composed of a plurality of needle-shaped recessed portions formed on the surface of the mold. The nozzle was pressed against the mold with a pressure of 1.372 x 10⁴Pa (0.14 kgf/cm²) at a position 2 mm in the direction opposite to the second row with respect to the first row. While pressing the nozzle, the Z-axis was controlled so that the fluctuation of the pressing pressure was kept within ±0.490 × 10⁴Pa (0.05kgf/cm²), and the nozzle was allowed to move at 0.5 mm/sec to the direction perpendicular to the length direction of the opening portion. At the same time, using the liquid-supplying device, the solution containing an inactivated Japanese encephalitis virus was released from the opening portion at 0.15 µL/sec for 20 seconds. The nozzle was passed through the hole patterns of a two-dimensionally arranged plurality of needle-shaped recessed portions, and then, the movement of the nozzle was then terminated at a position 2 mm apart from the hole patterns. Thereafter, the nozzle was separated from the mold.

The mold filled with the solution containing an inactivated Japanese encephalitis virus was placed in a lid (box) at 23°C in a 45% environment, and was then dried. At this time, the solution containing an inactivated Japanese encephalitis virus was gradually dried, and after 180 minutes or more, the water content became 20% or less. Further, the drying means is not limited to the lid, and other means such as temperature/humidity control and air volume control may also be used.

### (Formation and drying of sheet portion)

As a support for forming a sheet portion, a polyethylene terephthalate (PET) sheet (175 µm), which was subjected to a hydrophilic plasma treatment using a cloud remover (Victor jvc) under the following conditions (gas used: O₂, gas pressure: 13 Pa, high frequency (RF) power: 100 W, irradiation time: 3 minutes, O₂ flow rate: SV250, target vacuum degree (CCG): 2.0 × 10⁻⁴Pa), was used. A solution for forming the sheet portion was applied onto the front and back surfaces of the treated PET, to result in a film thickness of 75 µm. On the other hand, the mold filled with the solution containing an inactivated Japanese encephalitis virus was fixed on a suction table by suction. The surface side of the PET coated with the solution for forming the sheet portion was placed, so that it was faced to the mold surface, and thereafter, the void between the PET and the mold, and the space of the PET on the side opposite to the mold, were further depressurized for 2 minutes. After completion of the depressurization, only the space of the PET on the side opposite to the mold was released to atmospheric pressure, so that the PET coated with the solution for forming the sheet portion and the mold were adhered to each other. After maintaining the contact state for 10 minutes, an integrated product obtained by adhesion of the PET and the mold was dried in an environment of 23°C and 45% (relative humidity).

### (Peeling step)

By carefully peeling the dried and solidified microneedle array from the mold, a microneedle array containing an inactivated Japanese encephalitis virus was formed. The present microneedle is composed of a frustum portion and a needle portion, the length L of the needle-shaped protrusion portion was about 800 µm, and the width of the basement was about 350 µm. The frustum portion had a truncated cone structure, in which the height was about 190 µm, the diameter of the upper bottom surface was about 350 µm, and the diameter of the lower bottom surface was about 700 µm. The number of needles was 109 needles, which were arranged with needle intervals of about 1 mm.

### < Test 1: Human clinical trial of Japanese encephalitis preventive agent >

### (Place of implementation)

### Hokkaido University Hospital

### (Research design)

Randomized comparison, open-label, active control, parallel group comparison

### (Subject)

39 Cases of healthy adults aged 20 to 35 years without having antibodies to Japanese encephalitis virus

The subjects were randomly assigned by block randomization to 3 groups of MNA-H, MNA-L, and SC, as 1 group consisting of 13 cases.

Exclusion criteria include the following situations: subjects exhibiting apparent fever (37.5°C or higher); subjects having, at an administration site, skin disease that may cause interference to administration; subjects with immunodeficiency disease (AIDS, primary immunodeficiency, etc.); subjects who have been vaccinated with a Japanese encephalitis vaccine in the past; subjects who have affected with Japanese encephalitis in the past; subjects who have had anaphylaxis due to other vaccinations; subjects having a history of convulsions in the past; subjects being affected with serious acute disease; subjects who have received an inactivated vaccine within 6 days before initiation of the administration; subjects who have received a live vaccine within 27 days before initiation of the administration; subjects who have received blood transfusion or have administered with a γ-globulin preparation within 90 days before initiation of the administration; subjects whose Japanese encephalitis virus neutralizing antibody titer was positive in an immunoserological test performed upon screening; subjects in whom any of HIV antigen/antibody, HBs antigen, HCV antibody, TP antibody, and HTLV-1 antibody was positive in an infectious disease test upon screening; (female) subjects who are pregnant or are likely to be pregnant, who hoped to be pregnant during the present research period, or who are during breastfeeding or are difficult to contracept by using mechanical contraceptives such as uterine interior fittings or barrier method (pessary or condom), and by combining them; and subjects who were determined by doctor that participation to the present research was inappropriate.

### (Test method)

MNA-H: containing 0.63 µg of inactivated Japanese encephalitis virus
MNA-L: containing 0.25 µg of inactivated Japanese encephalitis virus
SC: containing 2.5 µg of inactivated Japanese encephalitis virus (Jevik V, General Incorporated Association, Research Institute for Microbial Diseases, Osaka University)

In the MNA-H group and the MNA-L group, using an applicator, one microneedle array was administered to the upper arm of each subject via patch administration for the first administration (week 0), and thereafter, another microneedle array was administered thereto 3 weeks after the first administration. In the SC group, Jevik V was subcutaneously injected into the upper arm once for the first time (0 week), and thereafter, Jevik V was subcutaneously injected therein once 3 weeks after the first administration.

### (Inspection items)

For evaluation of efficacy, an immunoserological test was carried out upon screening, at an initial administration (week 0), 2 weeks after the first administration, at a second administration (3 weeks after the first administration), 6 weeks after the first administration, and 27 weeks after the first administration. For evaluation of safety, a hematological test and a biochemical test were carried out upon screening, at an initial administration (week 0), 2 days after the first administration, 2 weeks after the first administration, at a second administration (3 weeks after the first administration), 6 weeks after the first administration, and 27 weeks after the first administration. In addition to the aforementioned observation periods, subjective and objective symptoms at the administration site were confirmed 15 weeks after the first administration.

### (Consideration for ethics)

This clinical study was a specific clinical study approved by the Hokkaido University Hospital Accredited Clinical Research Review Board, and the content of the study was fully explained at the time of implementation, and informed consent was obtained in writing.

### (Safety)

No significant adverse reactions (i.e. shock, anaphylaxis, acute disseminated encephalomyelitis, convulsions, thrombocytopenic purpura, and encephalitis/encephalopathy) were observed in this clinical study.

### (Evaluation of efficacy)

The neutralizing antibody contained in the serum of each subject was measured using the CPE microplate method as described below.

### Vero cell seeding:

Trypsin-treated Vero cells were adjusted to 2.0 × 10⁵ cells/mL using an EMEM medium for cell culture (Gibco, +10% FBS, +kanamycin), and were then used as a cell suspension. The cell suspension was inoculated in an amount of 100 µL each on a cell culture microplate (IWAKI 96-well plate flat, AGC TECHNO GLASS) and was then cultured in a CO₂ incubator for 1 day (37°C, 5% CO₂).

### Preparation of serum sample:

The subject's serum was inactivated by heating at 56°C for 30 minutes. On a microplate for serum dilution (IWAKI 96-well plate round, AGC TECHNO GLASS), 2-fold serial dilution series from 10 times to 10240 times were produced using an EMEM medium (Gibco, +2% FBS), so that all wells contained 20 µL.

### Neutralization reaction:

As an attacking virus, a Japanese encephalitis virus Beijing strain (National Institute of Infectious Diseases) prepared to be 4.0 × 10³ pfu/mL using an EMEM medium (Gibco, +2% FBS) was used, and this virus was then added in an amount of 20 µL/well into the above-described microplate for serum dilution, and was then subjected to a neutralization reaction in a CO₂ incubator for 1.5 hours (37°C, 5% CO₂).

### Vero cell infection experiment:

The above-described mixed solution of the neutralized serum and the attacking virus was inoculated in an amount of 25 µL/well into the above-described cell culture microplate and was then cultured in a CO₂ incubator for days (37°C, 5% CO₂).

### Cell immobilization and staining:

A formalin solution was added in an amount of 40 µL/well into the above-described cell culture microplate, and was then left at rest for 2 hours or more. Thereafter, the formalin solution was removed, and the plate was then washed with tap water. After that, a 0.02% methylene blue stain solution was added in an amount of 100 µL/well, and the obtained mixture was then left at rest for 1 hour or more, so that the cells were stained. A common logarithm of the highest dilution rate of the above-described serum that prevented CPE caused by the attacking virus by 50% or more was defined to be a neutralizing antibody titer. When the dilution rate was less than 10 times, it was treated as 5 (common logarithm: 0.7).

**[Table 1]**

| | Administration group | Solution I | | | | Solution II |
|---|---|---|---|---|---|---|
| | | Inactivated Japanese encephalitis virus | Suc | GluNa | Tw | HES |
| Example 1 | MNA-L | 0.25 µg | 750 µg | 208 µg | 1.7 µg | 32000 µg |
| Example 2 | MNA-H | 0.64 µg | 750 µg | 208 µg | 1.7 µg | 32000 µg |

### (Results of Test 1)

Thirty-nine subjects satisfied the criteria and were randomized. Before administration, the neutralizing antibody titer in serum was 0.7 in all of the SC group, MNA-L group, and MNA-H group (Table 2). Subjects whose neutralizing antibody titer in serum became 1.3 or more were defined as positive conversion, and the positive conversion rate was calculated. Two weeks after the first administration, 100% of the MNA-H group turned positive, but the SC group had a positive conversion percentage of 46%, and the MNA-L group had a positive conversion percentage of 54%. Six weeks after the first administration, all of the groups had a positive conversion percentage of 100%.

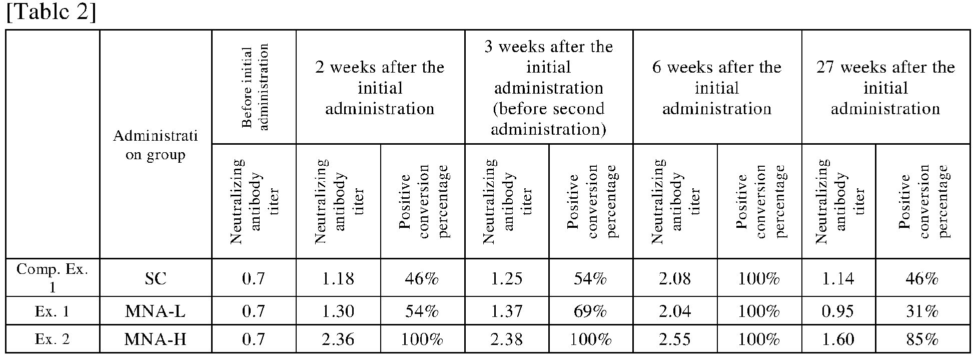

### (Measurement of dose of inactivated Japanese encephalitis virus in each subject)

A Japanese encephalitis virus envelope protein-specific IgG antibody (General Incorporated Association, Research Institute for Microbial Diseases, Osaka University) was 3100-fold diluted with an immobilization buffer (0.05 mol/L bicarbonate buffer), and the diluted solution was then added in an amount of 50 µL each into wells to be used of a 96-well Microlon half area plate. The plate was left at rest at 4°C overnight or longer, so as to prepare a solid phase plate.

As for analyte, a sample solution was prepared by dissolving the microneedle array after the administration in a dilution buffer (DPBS + 0.1% BSA + 0.5% Tw). 30 µL of a standard antigen (General Incorporated Association, Research Institute for Microbial Diseases, Osaka University) was added to 218 µL of the dilution buffer to prepare a stock solution of a standard solution. The stock solution was diluted 10 times, 16.6 times, 50 times, 100 times, and 500 times, to prepare individual standard solutions.

The solid phase plate was washed 3 times with PBS-T (DPBS + 0.05% Tween 20). Using a 12-channel micropipette, 100 µL each of the solution was transferred from a round bottom plate for addition that contained the sample solution and the standard solution to the same position on the solid phase plate, and was then reacted at 37°C for 60 minutes. After completion of the reaction, the solid phase plate was washed 4 times with PBS-T, and 100 µL each of a solution prepared by 11000-fold diluting an HRP-labeled Japanese encephalitis virus envelope protein-specific IgG antibody (General Incorporated Association, Research Institute for Microbial Diseases, Osaka University) with FBS, to which PBS-T was added to 10% of the FBS, was added. The obtained mixture was reacted at 37°C for 60 minutes. After completion of the reaction, the plate was washed with PBS-T, 80 µL each of TMB was then added to all wells, and a reaction was then carried out at room temperature for 15 minutes under light interception. After 15 minutes had passed, 80 µL each of a reaction termination solution (0.5 mol/L H₂SO₄) was added to all of the wells, and the absorbance at 450 nm (reference: 620 nm) was then measured using a plate reader.

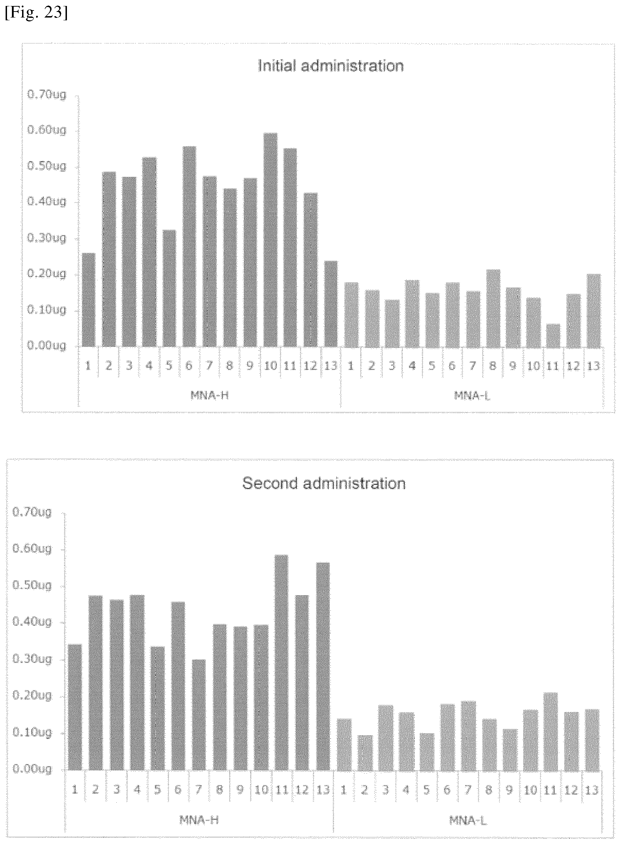

The results obtained by measuring the applied dose of the inactivated Japanese encephalitis virus to each subject as described above are shown in Fig. 23.

### < Test 2: Neutralizing antibody production test involving administration of Japanese encephalitis preventive agent to animals > (Vaccine administration test to mice)

Mice (Balb/c (CLEA Japan, Inc.), female, 7 weeks old) were purchased and were then acclimatized for 1 week. Thereafter, a Japanese encephalitis preventive agent administration test was carried out on the mice. As an initial administration, 100 µL of Jevik V, which had been appropriately diluted, was subcutaneously administered into the back of the neck of the mice. After that, the mice were raised for 15 days, and then, as a second administration, the back surface of the mice in the microneedle array group was dehaired under anesthesia, and the microneedle array (whose prescription is shown in Table 3) punctured the back of the mice for 10 minutes, while the skin was stretched. In the subcutaneous injection group, 100 µL of appropriately diluted Jevik V was subcutaneously administered into the back of the neck of the mice. All tests were performed on 4 mice.

### (Evaluation of efficacy)

After completion of the administration, the mice were raised for 1 week, and were then subjected to laparotomy under anesthesia, and thereafter, blood was collected from the posterior vena cava. The collected blood that had been left at rest at room temperature was centrifuged, and a supernatant was then collected to obtain serum. The amount of the antibody (IgG) specific to Japanese encephalitis virus that was contained in the obtained serum was measured by the following method.

### (Measurement of amount of IgG antibody produced)

A Japanese encephalitis vaccine was dispensed in an amount of 25 µL/well into a Microlon plate (flat bottom, 96 wells), and was then left at rest at 4°C overnight with a seal. After being left at rest, the resultant was washed 4 times with PBST (Gibco PBS + 0.05% Tw). Thereafter, a blocking solution (50 mmol/L Tris (pH 8.0) + 1% BSA) was added dropwise in an amount of 100 µL/well onto the plate, and the obtained mixture was then left at rest at room temperature for 30 minutes. After being left at rest, the reaction mixture was washed 4 times with PBST. After completion of the washing, a sample diluted about 10,000 times with a dilution solution (50 mmol/L Tris (pH 8.0) + 1% BSA + 0.05% Tw) was added dropwise in an amount of 100 µL/well onto the plate, and the obtained mixture was then left at rest at room temperature for 1 hour. After being left at rest, the reaction mixture was washed with PBST 4 times in the same manner as described above.

Subsequently, an HRP-labeled anti-mouse IgG antibody diluted with a dilution solution (500 times) was added dropwise in an amount of 100 µL/well onto the plate, and the obtained mixture was then left at rest at room temperature for 1 hour. After being left at rest, the reaction mixture was washed with PBST 4 times in the same manner as described above. Further, as a substrate reaction, TMB was added dropwise in an amount of 80 µL/well onto the plate, and the obtained mixture was then left at rest under light interception at room temperature for 15 minutes. Thereafter, a stop solution (1 mol/L HCl) was added dropwise in an amount of 80 µL/well onto the plate.

After completion of the dropwise addition, the absorbance at λ = 450 nm (reference: 620 nm) was measured, and the amount of the IgG antibody produced was expressed by the level of the absorbance. The average results obtained from the 4 mice are shown in Table 3.

**[Table 3]**

| | Solution I | | | | | Solution II | Amount of antibody generated |
|---|---|---|---|---|---|---|---|
| | 2nd administration method | Inactivated Japanese encephalitis virus | Suc | HES | GluNa | DEX/CS (7 : 3) | |
| Example 1 | Microneedle array | 0.015 µg | 3 µg | 6 µg | 0.3 µg | 7290 µg | 1.1290 |
| Example 2 | Microneedle array | 0.05 µg | 3 µg | 6 µg | 0.8 µg | 7290 µg | 1.7990 |
| Example 3 | Microneedle array | 0.15 µg | 3 µg | 6 µg | 2.5 µg | 7290 µg | 1.4705 |
| Comp. Ex. 1 | Subcutaneous injection | 0.015 µg | - | - | 1.2 µg | - | 0.5875 |
| Comp. Ex. 2 | Subcutaneous injection | 0.05 µg | - | - | 4 µg | - | 0.8963 |
| Comp. Ex. 3 | Subcutaneous injection | 0.15 µg | - | - | 12 µg | - | 0.8073 |
| Comp. Ex. 4 | Subcutaneous injection | 0.5 µg | - | - | 40 µg | - | 1.6290 |

### Reference Signs List

- 1: Microneedle array
- 2: Microneedle array
- 110: Microneedle
- 112: Needle portion
- 112A: Needle portion first layer
- 112B: Needle portion second layer
- 113: Frustum portion
- 116: Sheet portion
- 120: Layer that contains inactivated Japanese encephalitis virus
- 122: Layer that does not contain inactivated Japanese encephalitis virus
- W: Diameter (width)
- H: Height
- T: Height (thickness)
- 11: Original plate
- 12: Shaped portion
- 13: Mold
- 15: Needle-shaped recessed portion
- 15A: inlet portion
- 15B: Tip recessed portion
- 15C: Air vent hole
- D: Diameter
- 18: Mold complex
- 19: Gas permeable sheet
- 20: Base
- 22: Solution containing inactivated Japanese encephalitis virus
- 24: Solution containing at least one type of water-soluble polymer and disaccharide
- 29: Support
- 30: Tank
- 32: Pipe
- 34: Nozzle
- 34A: Lip portion
- 34B: Opening portion
- 36: Liquid-supplying device
- P1: Pressurizing force
- P2: Pressing force
- P3: Pressing force
- 40: Base material
- 50: Truncated cone
- 52: Cone
- D1: Diameter
- D2: Diameter
- L10: Pitch
- H1: Height
- H2: Height
- 61: X-axis driving portion
- 62: Z-axis driving portion
- 63: Nozzle
- 64: Liquid-supplying device
- 65: Suction table
- 66: Laser displacement meter
- 67: Load cell
- 68: Control mechanism
- 69: Mold

## Claims

1. A Japanese encephalitis preventive agent, comprising a microneedle array having a sheet portion and a plurality of needle portions present on the upper surface of the sheet portion, wherein the needle portions contain or carry an inactivated Japanese encephalitis virus.

2. The Japanese encephalitis preventive agent according to claim 1, wherein the inactivated Japanese encephalitis virus is administered at a dose of 0.01 µg to 2.5 µg/administration.

3. The Japanese encephalitis preventive agent according to claim 1 or 2, wherein the number of administrations is once or twice.

4. The Japanese encephalitis preventive agent according to any one of claims 1 to 3, wherein the inactivated Japanese encephalitis virus is administered twice at a dose of 0.01 µg to 0.22 µg/administration.

5. The Japanese encephalitis preventive agent according to any one of claims 1 to 3, wherein the inactivated Japanese encephalitis virus is administered once or twice at a dose of 0.23 µg to 1 µg/administration.

6. The Japanese encephalitis preventive agent according to any one of claims 1 to 5, wherein the microneedle array is an autolytic microneedle array, and the needle portions contain the inactivated Japanese encephalitis virus.

7. The Japanese encephalitis preventive agent according to any one of claims 1 to 6, wherein the needle portions comprise at least one type of a water-soluble polymer and a disaccharide, an inactivated Japanese encephalitis virus, and a surfactant, and the sheet portion comprises at least one type of a water-soluble polymer and a disaccharide.

8. The Japanese encephalitis preventive agent according to any one of claims 1 to 7, wherein the neutralizing antibody titer 2 weeks after the initial administration is 1.0 or more; provided that the neutralizing antibody titer is herein defined to be a common logarithm of the highest dilution rate of a serum that suppressed 50% or more of the cytopathic effect of the Japanese encephalitis virus, when 20 µL of a serum sample obtained by serially diluting a serum collected from a subject 2 weeks after administration of the Japanese encephalitis preventive agent is allowed to react with 80 pfu of Japanese encephalitis virus at 37°C and in 5% CO₂ for 1.5 hours, thereafter, 25 µL of the mixture obtained after completion of the reaction is infected into 2.0 x 10⁴ Vero cells, and thereafter, the obtained mixture are cultured at 37°C and in 5% CO₂ for 6 days.

9. A Japanese encephalitis vaccine agent, comprising a microneedle array having a sheet portion and a plurality of needle portions present on the upper surface of the sheet portion, wherein the needle portions contain or carry an inactivated Japanese encephalitis virus.

10. The Japanese encephalitis vaccine agent according to claim 9, wherein the inactivated Japanese encephalitis virus is administered at a dose of 0.01 µg to 2.5 µg/administration.

11. The Japanese encephalitis vaccine agent according to claim 9 or 10, wherein the number of administrations is once or twice.

12. The Japanese encephalitis vaccine agent according to any one of claims 9 to 11, wherein the inactivated Japanese encephalitis virus is administered twice at a dose of 0.01 µg to 0.22 µg/administration.

13. The Japanese encephalitis vaccine agent according to any one of claims 9 to 11, wherein the inactivated Japanese encephalitis virus is administered once or twice at a dose of 0.23 µg to 1 µg/administration.

14. The Japanese encephalitis vaccine agent according to any one of claims 9 to 13, wherein the microneedle array is an autolytic microneedle array, and the needle portions contain the inactivated Japanese encephalitis virus.

15. The Japanese encephalitis vaccine agent according to any one of claims 9 to 14, wherein the needle portions comprise at least one type of a water-soluble polymer and a disaccharide, an inactivated Japanese encephalitis virus, and a surfactant, and the sheet portion comprises at least one type of a water-soluble polymer and a disaccharide.

16. The Japanese encephalitis vaccine agent according to any one of claims 9 to 15, wherein the neutralizing antibody titer 2 weeks after the initial administration is 1.0 or more; provided that the neutralizing antibody titer is herein defined to be a common logarithm of the highest dilution rate of a serum that suppresses 50% or more of the cytopathic effect of the Japanese encephalitis virus, when 20 µL of a serum sample obtained by serially diluting a serum collected from a subject 2 weeks after administration of the Japanese encephalitis vaccine agent is allowed to react with 80 pfu of Japanese encephalitis virus at 37°C and in 5% CO₂ for 1.5 hours, thereafter, 25 µL of the mixture obtained after completion of the reaction is infected into 2.0 x 10⁴ Vero cells, and thereafter, the obtained mixture are cultured at 37°C and in 5% CO₂ for 6 days.
